# EUROPÄISCHE PATENTANMELDUNG

(11) **EP 4 847 120 A1**
(43) Veröffentlichungstag der Anmeldung: **02.12.2026**
(21) Anmeldenummer: 26180393.6
(22) Anmeldetag: 21.05.2026
(51) Int. Cl.: B01D 61/08, A61M 1/16, B01D 61/04

(54) **VERFAHREN UND SYSTEM ZUR ÜBERWACHUNG EINES SOLEBEHÄLTERS EINER UMKEHROSMOSEANLAGE**

(30) Priorität: 28.05.2025 DE 102025121012
(71) Anmelder: B. Braun Avitum AG, 34212 Melsungen (DE)
(72) Erfinder: LIEB, Nino, 34117 Kassel (DE); ODERNHEIMER, Philipp, 34212 Melsungen (DE)
(74) Vertreter: Tergau & Walkenhorst Intellectual Property GmbH

(57) **Zusammenfassung**

Verfahren zur Überwachung einer Salzfüllung eines Solebehälters (100) einer Umkehrosmoseanlage (130), dadurch gekennzeichnet, dass das Verfahren umfasst:
- Messen mindestens eines Messwertes mindestens einer Messgröße einer wässrigen Flüssigkeit im Solebehälter (100) mittels mindestens eines Sensors (101),
- optional Übermitteln des mindestens einen Messwerts an mindestens eine Auswerteeinheit (102), und
- Verarbeiten des mindestens einen Messwertes, optional in der mindestens einen Auswerteeinheit (102),

wobei der Sensor (101) ein Drucksensor (1011) ist, und das Verfahren umfasst:
- Messen eines Wasserdrucks P_{Drucksensor} der wässrigen Flüssigkeit im Solebehälter (100) mittels des Drucksensors (1011),
- Übermitteln des Wasserdrucks P_{Drucksensor} an die Auswerteeinheit (102), und
- Verarbeiten des Wasserdrucks P_{Drucksensor} in der Auswerteeinheit (102),

wobei das Verarbeiten des Wasserdrucks P_{Drucksensor} die Berechnung eines Wasserverdrängungsdrucks P_{Wasserverdrängung} umfasst, zur Bestimmung einer Salzfüllung des Solebehälters (100).

## Beschreibung

Die Erfindung betrifft die Überwachung der Salzfüllung eines Solebehälters einer beziehungsweise für eine Umkehrosmoseanlage, insbesondere im Rahmen der Wasseraufbereitung für Dialyseverfahren wie der Hämodialyse.

Für die Wasseraufbereitung mit hohen Anforderungen an die Wasserreinheit, beispielsweise im vder Umkehrosmoseeinheit mit Membran in der Regel mindestens eine Vorbehandlungseinheit, mit der Wasser zunächst vorbehandelt wird, bevor es der Umkehrosmoseeinheit zur weiteren Aufreinigung mittels der Membran zugeführt wird. Die Vorbehandlung schütz und entlastet die Umkehrosmoseeinheit. Sie ist für einen problemlosen Betrieb der Umkehrosmoseeinheit elementar.

Die Vorbehandlung umfasst die Wasserenthärtung mit einem dafür in der Vorbehandlungseinheit vorgesehen Enthärter, welche ein Ionenaustauscher-Material, beispielsweise ein kationisches Harz, enthält. Bei der Enthärtung wird Wasser mit dem mit Kationen, in der Regel Natriumionen, beladenen Ionenaustauscher-Material in Kontakt gebracht. Dabei werden dem Wasser Härtebildner (insbesondere zweiwertige Kalzium- und Magnesiumionen) entzogen und Natriumionen vom Ionenaustauscher-Material an das Wasser abgegeben. Die Härtebildner sättigen dabei nach und nach das Ionenaustauscher-Material, während diesem die Natriumionen entzogen werden.

Um die Funktionsfähigkeit der Vorbehandlungseinheit zu erhalten, muss daher in regelmäßigen Abständen das Ionenaustauscher-Material regeneriert werden. Hierzu wird das Ionenaustauscher-Material mit einer Salzlösung, in der Regel einer Kochsalz- oder NaCl-Lösung, beschickt, um Härtebildner aus dem Material zu verdrängen und durch Natriumionen zu ersetzen.

Zur Herstellung einer Salzlösung für die Regeneration werden Salz und Wasser in einen Solebehälter oder Salztank gegeben. Üblicherweise wird dabei genügend Salz für mehrere Regenerationen im Behälter vorgelegt. Im Behälter kann sich das Salz, beispielsweise in Tablettenform, auf einem Trägersieb oder in einem am Behälterrand befestigten oder aufgespannten wasserdurchlässigen Netz oder Salzsack befinden. Wird der Behälter nun mit Wasser befüllt, kann sich eine gesättigte Salzlösung bilden, die dann zur Regeneration des Ionentauscher-Materials durch die Vorbehandlungseinheit geleitet wird. Für die nächste Regeneration wird der Behälter dann erneut mit Wasser gefüllt. Durch die Herstellung und Entnahme von Salzlösung nimmt bei jeder Regeneration die noch im Solebehälter verbleibende Salzmenge ab, und das Salz wird aufgebraucht.

Um eine erfolgreiche Regeneration des Ionenaustauscher-Materials der Vorbehandlungseinheit zu gewährleisten und deren Funktionsfähigkeit zu erhalten, muss also sichergestellt werden, dass der Behälter rechtzeitig wieder mit Salz befüllt wird. Es soll weder versehentlichen eine nicht hinreichend konzentrierte Salzlösung hergestellt und bei der Regeneration eingesetzt werden, noch sollen im Betriebsablauf unnötige Unterbrechungen erforderlich werden, beispielsweise wenn eine Regeneration mangels hinreichender Salzfüllung des Behälters nicht zum gewünschten Zeitpunkt durchgeführt werden kann.

Bisher musste der Solebehälter in regemäßigen Abständen durch eine Sichtkontrolle auf seinen Salzgehalt geprüft werden. Bei der Sichtprüfung wird beispielsweise überprüft, ob das Salz noch bis zu einer am Behälter angebrachten Markierung reicht. Nachteile der Sichtprüfung umfassen das ungenaue Ergebnis und den manuelle Charakter der Prüfung, der Personalressourcen bindet, was gerade im Klinikbereich nicht wünschenswert ist.

Aus der DE102013011752A1 ist auch die Möglichkeit bekannt, den Solebehälter auf einer Waage zu platzieren. Gemäß DE102013011752A1 kann so der Füllstand des Solebehälters bzw. das Restvolumen von Salzen im Solebehälter überwacht und das enthaltene Solevolumen angezeigt werden. Diese Möglichkeit ist dahingehend aufwändig, dass der Solebehälter baulich auf der Waage platziert werden muss. Die Überwachung der Salzmasse im Solebehälter mittels Waage ist nur eingeschränkt möglich und gegebenenfalls recht ungenau. Die Messung kann leicht verfälscht werden, etwa wenn ein Gegenstand (bspw. ein Werkzeug oder Salzsack) auf dem Tank abgelegt wird, was die Unsicherheit des Verfahrens bzw. die Anfälligkeit gegenüber äußeren Störeinflussen erhöht.

Es besteht also ein Bedarf an einer verbesserten Überwachung des Salzfüllstandes in Solebehältern.

Aufgabe der Erfindung ist es, zumindest einen der oben genannten Nachteile zu überwinden. Insbesondere zielt die Erfindung darauf ab, ein verbessertes Verfahren und System zur Überwachung der Salzfüllung eines Solebehälters einer beziehungsweise für eine Umkehrosmoseanlage bereitzustellen, und die automatische Überwachung der Salzfüllung zu ermöglichen.

### Kurzzusammenfassung

Die Aufgabe wird durch ein Verfahren nach Anspruch 1 und ein System nach Anspruch 8 gelöst. Die erfindungsgemäße Überwachung der Salzfüllung basiert auf einer Sensormessung, bevorzugt einer Drucksensormessung.

Dementsprechend wird in einem ersten Aspekt ein Verfahren zur Überwachung einer Salzfüllung eines Solebehälters einer Umkehrosmoseanlage bereitgestellt, dadurch gekennzeichnet, dass das Verfahren umfasst:
- Messen mindestens eines Messwertes mindestens einer Messgröße einer wässrigen Flüssigkeit im Solebehälter mittels mindestens eines Sensors,
- optional Übermitteln des mindestens einen Messwerts an mindestens eine Auswerteeinheit, und
- Verarbeiten des mindestens einen Messwertes, optional in der mindestens einen Auswerteeinheit,
wobei optional der Sensor ausgewählt ist aus mindestens einem Drucksensor, mindestens einem Füllstandsensor und Kombinationen davon. Bevorzugt ist der Sensor ein Drucksensor. Das Verfahren kann insbesondere das Messen eines Wasserdrucks P_{Drucksensor} der wässrigen Flüssigkeit im Solebehälter mittels eines Drucksensors umfassen. Das Verfahren kann weiterhin das Übermitteln des Wasserdrucks P_{Drucksensor} an eine Auswerteeinheit und das Verarbeiten des Wasserdrucks P_{Drucksensor} in der Auswerteeinheit umfassen. Das Verarbeiten des Wasserdrucks P_{Drucksensor} umfasst bevorzugt die Berechnung eines Wasserverdrängungsdrucks P_{Wasserverdrängung} , zur Bestimmung einer Salzfüllung des Solebehälters.

Gemäß einem zweiten Aspekt wird eine Vorrichtung, genauer ein System, zur Überwachung einer Salzfüllung eines Solebehälters für eine Umkehrosmoseanlage bereitgestellt, dadurch gekennzeichnet, dass das System mindestens einen Sensor zum Messen mindestens eines Messwertes mindestens einer Messgröße einer gegebenenfalls im Solebehälter enthaltenen wässrigen Flüssigkeit umfasst, wobei der mindestens einen Sensor zum Messen des mindestens eines Messwertes am oder im Solebehälter angeordnet ist, und
wobei optional das System weiter eine Auswerteeinheit und/oder den Solebehälter umfasst. Das System umfasst bevorzugt mindestens einen Drucksensor zum Messen eines Wasserdrucks P_{Drucksensor} einer gegebenenfalls im Solebehälter enthaltenen wässrigen Flüssigkeit.

Das erfindungsgemäße Verfahren und System ermöglichen in vorteilhafter Weise ein automatische Überwachung der Salzfüllung des Solebehälters. Die Salzfüllung bzw. der Salzfüllstand des Solebehälters kann sehr zuverlässig überwacht werden. Gegenüber den im Stand der Technik üblichen manuellen Verfahren wird eine höhere Genauigkeit und Sicherheit erreicht, Ablesefehler und Versäumen der rechtzeitigen Ablesung können vermieden und zusätzliche Aussagen getroffen werden. Die Umkehrosmoseeinheit, insbesondere deren Membran, und somit letztlich die gesamte Umkehrosmoseanlage können sehr wirksam vor Beschädigungen, insbesondere Verkalkungen, durch unzureichend vorgereinigtes Wasser geschützt werden.

Ausfälle der Anlage und Standzeiten sowie damit verbundene Kosten können verhindert und die Qualität, insbesondere die Zuverlässigkeit, der Versorgung mit hochreinem Wasser erhöht werden. Dies ist gerade im medizinischen Kontext, bspw. bei der Dialyse, wichtig. Die Dialyseanlage muss zuverlässig funktionsfähig sein und es muss hinreichend reines Wasser zur Verfügung stehen, um die Patientenversorgung und -sicherheit nicht zu gefährden. Hier wird durch die Erfindung eine deutliche Verbesserung erreicht.

Gleichzeitig ist der apparative Aufwand vergleichsweise gering und verwendete Sensoren, insbesondere Drucksensoren, sowie weitere Komponenten des Systems können auch in übliche Vorrichtungsdesigns oder bereits bestehende Vorrichtungen integriert werden. Die erfindungsgemäße Überwachung der Salzfüllung ist vorteilhaft auch dahingehend sehr flexibel, dass die Messung bei Bedarf sehr genau ausgestaltet werden kann, es gleichzeitig aber auch möglich ist, die Salzfüllung mit einer für die Praxis gut geeigneten Genauigkeit zu messen, wenn eine oder mehrere vereinfachende Annahmen, bspw. bezüglich der Solebehältergeometrie und/oder der Temperatur, getroffen werden.

### Detaillierte Beschreibung der Erfindung

Weitere Aspekte, Ausführungsformen und Vorteile der Erfindung ergeben sich auch aus der nachfolgenden detaillierten Beschreibung und den Beispielen, sowie der Diskussion der beigefügten Figuren. Dabei zeigt:
- Fig. 1A: Solebehälter
- Fig. 1B: Solebehälter mit Drucksensor
- Fig. 2: Solebehälter mit Volumenstromsensor
- Fig. 3: Vorbehandlungseinheit mit Absperrmöglichkeit
- Fig. 4: Auswerteeinheit mit Kommunikationsmöglichkeit zur Umkehrosmoseeinheit
- Fig. 5: Solebehälter mit Drucksensor und Temperatursensor
- Fig. 6: Solebehälter mit Füllstandsensor
- Fig. 7: Umkehrosmoseanlage
- Fig. 8: Druckmessung während der Entnahme eines Salzsacks aus einer gesättigten Salzlösung im Solebehälter und der Rückgabe in die Salzlösung
- Fig. 9: Druckmessung während der Regeneration und dem Wiederauffüllen des Solebehälters

**In** der Beschreibung der Erfindung für das Verfahren offenbarte Aufgaben, Merkmale, Eigenschaften und Vorteile sind sinngemäß auf das System übertragbar und umgekehrt.

### A. Verfahren

**In** einem ersten Aspekt stellt die Erfindung ein Verfahren zur Überwachung einer Salzfüllung eines Solebehälters einer Umkehrosmoseanlage bereit, dadurch gekennzeichnet, dass das Verfahren umfasst:
- Messen mindestens eines Messwertes mindestens einer Messgröße einer wässrigen Flüssigkeit im Solebehälter mittels mindestens eines Sensors,
- optional Übermitteln des mindestens einen Messwerts an mindestens eine Auswerteeinheit, und
- Verarbeiten des mindestens einen Messwertes, optional in der mindestens einen Auswerteeinheit.

Die Verwendung einer Auswerteinheit ist bevorzugt. Vorteilhafterweise kann die Überwachung mit dem erfindungsgemäßen Verfahren teil- oder vollautomatisch erfolgen. Das Verfahren kann auch unter Verwendung des erfindungsgemäßen Systems durchgeführt werden.

Als Sensor kann insbesondere ein Drucksensor und/oder ein Füllstandsensor eingesetzt werden. Bevorzugt wird zumindest ein Drucksensor eingesetzt. Auch der Einsatz mehrerer Sensoren ist möglich, und verschiedene Sensortypen können in vorteilhafter Weise miteinander kombiniert werden, wie auch aus der nachfolgenden Beschreibung ersichtlich.

In vorteilhafter Weise kann mit dem erfindungsgemäßen Verfahren, insbesondere bei Verwendung eines Drucksensors, die Salzfüllung des Solebehälters beispielsweise auf mindestens 2,5 kg genau, mindestens 2 kg genau, mindestens 1 kg genau, oder mindestens 500 g genau bestimmt werden. Bevorzugt erfolgt die Bestimmung auf 1 kg bis 2 kg genau, oder auf 1 kg genau. Die im Solebehälter befindliche Salzmasse kann bspw. auf mindestens 10% genau oder mindestens 8%, mindestens 5% oder mindestens 2% genau bestimmt werden. Bevorzugt wird die Masse auf mindestens 5% genau bestimmt.

### A.1. Messung mit Drucksensor

Bevorzugt kommt im Verfahren mindestens ein Drucksensor zum Einsatz. Gemäß einer bevorzugten Ausführungsform, umfasst das Verfahren entsprechend:
- Messen eines Wasserdrucks P_{Drucksensor} der wässrigen Flüssigkeit im Solebehälter mittels des Drucksensors,
- optional Übermitteln des Wasserdrucks P_{Drucksensor} an eine Auswerteeinheit, und
- Verarbeiten des Wasserdrucks P_{Drucksensor}, optional in der Auswerteeinheit.

### A.1.1. Messen des Wasserdrucks P_{Drucksensor}

Der Wasserdruck (P_{Drucksensor}) der wässrigen Flüssigkeit im Solebehälter wird mittels Drucksensor gemessen.

Bei der wässrigen Flüssigkeit kann es sich um Reinwasser, insbesondere Betriebswasser, oder um eine Salzlösung, insbesondere eine gesättigte Salzlösung handeln. Die Salzlösung bildet sich im Behälter nach Zugabe von Wasser und Salz, wobei üblicherweise das Salz bei der Zugabe des Wassers bereits im Behälter vorhanden ist, beispielsweise in Form von Salztabletten.

Der Einsatz eines Drucksensors ist apparativ recht unaufwändig, und der Drucksensor kann mit geringem Platzbedarf am Solebehälter angeordnet werden. Das Aufbocken des Solebehälters auf einen Unterbau ist nicht erforderlich. Mit guter Genauigkeit und Flexibilität sind eine Vielzahl von Aussagen bezüglich der Salzfüllung möglich, wie auch nachfolgend weiter erläutert.

### A.1.2. Optionales Übermitteln des Wasserdrucks P_{Drucksensor} an eine Auswerteeinheit

Der gemessene Wasserdruck P_{Drucksensor} wird dann vorzugsweise an eine Auswerteeinheit übermittelt. Die Übermittlung kann dabei über ein Kommunikationsmittel wie auch unten für das System beschrieben erfolgen. Der Drucksensor kann beispielsweise ein elektrisches Signal an die Auswerteeinheit übermitteln. Die Verwendung einer Auswerteeinheit vereinfacht die Durchführung des Verfahrens und unterstützt dessen Automatisierbarkeit bei. Berechnungsfehler können vermieden werden. Entsprechend können alle nachfolgend Beschriebenen weiteren Ausführungsformen insbesondere unter Verwendung einer Auswerteeinheit verwirklicht werden.

### A.1.3. Verarbeiten des Wasserdrucks P_{Drucksensor}

Der mittels Drucksensor gemessene Wert für den Wasserdruck P_{Drucksensor} wird verfahrensgemäß weiterverarbeitet, bevorzugt in der Auswerteinheit. Bei der Verarbeitung kann auf verschiedene Formeln und Gleichungen, insbesondere wie nachfolgend näher erläutert, zurückgegriffen werden.

Neben dem gemessene Wasserdruck P_{Drucksensor} kann insbesondere der Wasserdruck herangezogen werden, der für das bei der Messung von P_{Drucksensor} im Solebehälter befindliche Flüssigkeitsvolumen zu erwarten ist, wenn keine Volumenverdrängung durch Salz im Behälter erfolgt. In einer Ausführungsform kann der erwartete Wasserdruck nach der folgenden Formel berechnet werden: ${P}_{Wasserdruck \left(erwartet\right)} \left[kg/\left({s}^{2}*m\right)\right] = \left(ρ \left[kg/{m}^{3}\right]*V \left[{m}^{3}\right]*g \left[m/{s}^{2}\right]\right) / A \left[{m}^{2}\right] ,$ wobei ρ die Dichte der wässrigen Flüssigkeit, V das Volumen der gemessenen Flüssigkeit, g der Wert für die Erdbeschleunigung und A die Grundfläche des Solebehälters ist.

In einer bevorzugten Ausgestaltung wird für die weitere Berechnung auf den theoretischen Wasserdruck P_{Wasserdruck (theoretisch)} oder den mittels eines gemessenen Wasservolumens bestimmten Wasserdruck P_{Wasserdruck} zurückgegriffen. Auf diese wird nachfolgend weiter eingegangen.

### A.1.3.1. Erklärungen zum theoretischen Wasserdruck P_{Wasserdruck (theoretisch)} und zum mittels eines gemessenen Wasservolumens bestimmten Wasserdruck P_{Wasserdruck}

Aus Gründen der Übersichtlichkeit werden die folgenden Erklärungen zum theoretischen Wasserdruck (P_{Wasserdruck (theoretisch)}) und zum mittels eines gemessenen Wasservolumens bestimmten Wasserdruck (P_{Wasserdruck}) der weiteren Beschreibung vorangestellt. Auf die Erklärungen wird nachfolgend dann bei Bedarf verwiesen.

### a) Der theoretische Wasserdruck P_{Wasserdruck (theoretisch)}

Der theoretische Wasserdruck P_{Wasserdruck (theoretisch)} kann beispielsweise als der Druck beschrieben werden, der für ein Volumen wässriger Flüssigkeit erwartet wird, wenn sich kein Salz im Solebehälter befindet, und es somit zu keiner Volumenverdrängung durch Salz im Behälter kommt. Er kann mit folgender Gleichung bestimmt werden: ${p}_{wasserdruck \left(theoretisch\right)} \left[\frac{kg}{{s}^{2} ∗ m}\right] = \frac{{ρ}_{Wasser} \left[\frac{kg}{{m}^{3}}\right] ∗ {V}_{wasser} \left[{m}^{3}\right] ∗ g \left[\frac{m}{{s}^{2}}\right]}{{A}_{Solebeh ä lter \left[{m}^{2}\right]}}$ wobei ρWasser eine Dichte der wässrigen Flüssigkeit im Solebehälter, A_{Solebehälter} die Grundfläche des Solebehälters, g der Wert für die Erdbeschleunigung (ansetzbar insbesondere mit 9,80665 m/s²) und V_{Wasser} ein festgesetzter Wasservolumenwert ist.

Der für V_{Wasser} festgesetzte und somit vorgegebene Wasservolumenwert ist bevorzugt in der Auswerteeinheit hinterlegt. Wird der theoretische Wasserdruck für weitere Berechnungen eingesetzt, sollte entsprechend das Wasservolumen, mit dem der Solebehälter bei der Druckmessung befüllt ist, zumindest im Wesentlichen dem für den theoretischen Wasserdruck angesetzten Volumen V_{Wasser} entsprechen, also insbesondere nicht mehr als 10% oder nicht mehr als 5% von diesem abweichen. Es ist daher in der Praxis insbesondere zweckmäßig, für V_{Wasser} ein Einfüllwasservolumen anzusetzen. Die Druckmessung kann dann durchgeführt werden, wenn der Behälter mit dem Einfüllwasservolumen befüllt ist. Das Einfüllwasservolumen kann dabei dem Wasservolumen entsprechen, mit dem der Solebehälter zur Herstellung einer Salzlösung für die Regeneration befüllt wird, wenn er noch keine Flüssigkeit enthält. Anders gesagt kann das Einfüllwasservolumen als das Wasservolumen verstanden werden, das nötig ist, um den noch keine Flüssigkeit enthaltenden Behälter zum Durchführen einer Regeneration zu füllen.

Die Grundfläche A_{Solebehälter} des Solebehälters fließt in die Berechnung mit ein.

Die weitere Behältergeometrie kann berücksichtigt werden, der Einfluss der Gefäßform des Solebehälters ist aber typischerweise vernachlässigbar, so dass insbesondere bei den typischerweise als Solebehälter eingesetzten zylinderförmigen Behältern, wie Tanks oder Tonnen, mit hinreichender Genauigkeit des Ergebnisses von einem näherungsweise idealen Zylinder oder idealen Zylinder ausgegangen werden kann.

Die Dichte ρWasser der wässrigen Flüssigkeit im Solebehälter wird mit Hinblick auf die tatsächliche Dichte (beispielsweise durch Messung ermittelt) oder die erwartete Dichte der wässrigen Flüssigkeit bei der Druckmessung angesetzt.

Die erwartete Dichte der wässrigen Flüssigkeit ρ_{Wasser} kann in Abhängigkeit von der Verweildauer des Wassers im Solebehälter vor der Druckmessung ausgewählt werden, da das Verweilen im Behälter nach der Befüllung nach und nach zu einer Erhöhung der Salzkonzentration bis hin zur Sättigung führt. Dies wird im Folgenden weiter verdeutlicht.
(i) Als Dichte der wässrigen Flüssigkein ρ_{Wasser} kann die Dichte reinen Wassers, bevorzugt 998,2 kg/m³, ausgewählt werden, wenn die Druckmessung innerhalb von 0,5 h, bevorzugt innerhalb von 0,25 h, weiter bevorzugt innerhalb von 5 min oder unmittelbar nach dem Befüllen des Solebehälters mit einem Wasservolumen erfolgen, wenn der Solebehälter vor dem Befüllen mit dem Wasservolumen im Wesentlichen keine oder keine Flüssigkeit enthielt. Insbesondere bei einer Messung innerhalb von 15 min oder 5 min kann mit guter Genauigkeit der Berechnung davon ausgegangen werden, dass sich noch wenig Salz im Wasser gelöst hat.
(ii) Als Dichte der wässrigen Flüssigkein ρ_{Wasser} kann auch die Dichte einer gesättigten Salzlösung, bevorzugt 1202 kg/m³, ausgewählt werden, wenn die Druckmessung mindestens 1 h, bevorzugt mindestens 1,5 h, weiter bevorzugt mindestens 2 h nach dem Befüllen des Solebehälters mit einem Wasservolumen erfolgt, wenn der Solebehälter vor dem Befüllen mit dem Wasservolumen im Wesentlichen keine oder keine Flüssigkeit enthielt.

Der Ausdruck "im Wesentlichen keine Flüssigkeit" kann dabei bedeuten, dass etwaige bereits im Behälter enthaltene Flüssigkeit ein Volumen von nicht mehr als 10%, oder nicht mehr als 5%, des eingefüllten Wasservolumens hat.

Es kann auch unter Berücksichtigung der Kontaktzeit der Flüssigkeit mit Salz im Behälter (beispielsweise 0,75 h) vor der Messung zwischen dem Dichtewert reinen Wassers und dem Dichtewert einer gesättigten Salzlösung interpoliert werden, wieviel Salz sich bereits gelöst hat und somit welche Dichte bei der Messung vorliegt. Die Variante mit Interpolation ist aufwändiger, kann zu einem etwas ungenaueren Ergebnis führen, und ist somit weniger bevorzugt.

Enthält der Solebehälter vor dem Befüllen mit Wasser bereits ein Restflüssigkeitsvolumen (insbesondere ein Volumen von mehr als 10%, oder mehr als 5% des eingefüllten Wasservolumens), kann zur Erhöhung der Genauigkeit wie folgt vorgegangen werden:
(iii) Als Dichte der wässrigen Flüssigkein ρ_{Wasser} wird eine Mischdichte angesetzt, wenn die Druckmessung innerhalb von 0,5 h, bevorzugt innerhalb von 0,25 h, weiter bevorzugt innerhalb von 5 min oder unmittelbar nach dem Befüllen des Solebehälters mit Wasser erfolgt. Die Mischdichte kann mit folgender Formel berechnet werden: ρ_{Mischdichte} = (ρ_{a *} Vₐ + ρ_{b *} V_{b} +) / (Va + Vb), wobei ρa die Dichte von Wasser, Va das in den Behälter gefüllte Wasservolumen, ρb die Dichte der Restflüssigkeit, also in der Regel die Dichte einer gesättigten Salzlösung, und Vb das Restflüssigkeitsvolumen ist.
Kann aufgrund der Standzeit vor der Messung dagegen davon ausgegangen werden, dass auch bereits das in den Behälter gefüllte Wasservolumen nunmehr als gesättigte Salzlösung vorliegt, wird als Dichte der wässrigen Flüssigkein ρ_{Wasser} entsprechend die Dichte einer gesättigten Salzlösung, bevorzugt 1202 kg/m³, ausgewählt.

Der Einfluss der Temperatur auf die Dichte der wässrigen Flüssigkeit ρ_{Wasser} kann wie folgt berücksichtigt werden, wobei Ausführungsformen gemäß (i) - (iv) bei Bedarf auch kombiniert werden können:
(i) Eine Standarddichte wird als ρ_{Wasser} angenommen, bei der Temperatureinfluss auf die Dichte vernachlässigt. In der praktischen Umsetzung der Erfindung ist der Einfluss der Temperatur auf die weitere Berechnungsgenauigkeit gering und kann vernachlässigt werden. Die Verwendung einer Standarddichte ist bevorzugt und aufgrund ihrer Einfachheit vorteilhaft. Als Standarddichte reinen Wassers kann beispielsweise eine Dichte von 998,2 kg/m³ angenommen werden.
(ii) Es erfolgt eine Temperaturmessung im Solebehälter, bevorzugt über ein dafür im Solebehälter befindliches Mittel zur Temperaturmessung, insbesondere einen Temperatursensor. Die Temperatur kann an die Auswerteeinheit übermittelt werden. ρ_{Wasser} wird gemäß der gemessenen Temperatur korrigiert und/oder ausgewählt.
(iii) Es erfolgt eine Temperaturmessung, beispielsweise mittels eines Thermostats oder Temperatursensors, in der Nähe des Solebehälters, vorzugsweise im selben Raum und/oder in einem Abstand von höchstens 5 m, 3 m oder 1 m zum Solebehälter. Die Temperatur kann an die Auswerteeinheit übermittelt werden. ρ_{Wasser} wird gemäß der gemessenen Temperatur korrigiert und/oder ausgewählt. Diese Variante ist ebenfalls ausreichend genau, insbesondere nach längerer Standzeit der Flüssigkeit im Solebehälter, da sich über die Zeit die Temperatur an die Umgebungs- bzw. die Raumtemperatur angleicht.
(iv) In der Auswerteeinheit ist eine durchschnittliche Temperatur, bevorzugt eine durchschnittliche Raumtemperatur, hinterlegt, die zur Kompensation und/oder Auswahl von ρ_{Wasser} verwendet wird.

Die Dichten von Reinwasser bzw. Betriebswasser sowie die Dichten von wässrigen Salzlösungen, insbesondere gesättigten Salzlösungen (bspw. von Kochsalz bzw. NaCl) bei unterschiedlichen Temperaturen sind dem Fachmann bekannt und in der Literatur beschrieben. Beispielhafte Dichten für Reinwasser, die angesetzt werden können, sind: 10°C: 999,7 kg/m³, 12°C: 999,50 kg/m³, 25°C: 997,04 kg/m³, 30°C: 995,6 kg/m³.

In vorteilhafter Weise können Werte, bspw. für den theoretischen Wasserdruck, ρWasser, A_{Solebehälter}, g und/oder V_{Wasser}, in der Auswerteinheit, bevorzugt in einem persistenten Speicher der Auswerteinheit, hinterlegt sein. Dies vereinfacht die Auswertung und begünstigt die Automatisierung. Dem Fachmann wird aus den obigen Ausführungen auch unmittelbar klar sein, dass es zweckdienlich sein kann, mehrere alternative Werte, bspw. für den theoretischen Wasserdruck und ρWasser in der Auswerteinheit zu hinterlegen, auf die dann in Abstimmung mit den Druckmessbedingungen, insbesondere den Zeitpunkt nach Befüllung des Behälters mit Wasser und die daher anzusetzende Dichte, zugegriffen werden kann.

b) Der mittels eines gemessenen Volumens bestimmte Wasserdruck P_{Wasserdruck} Es ist auch eine Ausgestaltung möglich bei der, anders als für den theoretischen Wasserdruck (wo für V_{Wasser} wird ein Wasservolumenwert festgesetzt und somit vorgegeben wird), das jeweils in den Solebehälter eingefüllte Wasservolumen gemessen und das gemessen Wasservolumen V_{Wasser(gemessen)}, bevorzugt nach Übermittlung an die Auswerteeinheit, für die weitere Berechnung verwendet wird. Die Messung kann mittels Volumenstromsensor erfolgen, wie auch in der weiteren Beschreibung noch näher dargelegt. Durch den Einsatz des gemessen Wasservolumens kann die Genauigkeit der weiteren Berechnung erhöht und auch eine höhere Flexibilität hinsichtlich der Befüllung des Behälters erreicht werden, da das Volumen direkt durch Messung erhalten wird. Wir V_{Wasser(gemessen)} eingesetzt und der Wasserdruck so ermittelt, gilt: ${p}_{wasserdruck} \left[\frac{kg}{{s}^{2} ∗ m}\right] = \frac{{ρ}_{Wasser} \left[\frac{kg}{{m}^{2}}\right] ∗ {V}_{wasser} \left(gemessen\right) \left[{m}^{2}\right] ∗ g \left[\frac{m}{{s}^{2}}\right]}{{A}_{Solebeh ä lter \left[{m}^{2}\right]}} .$

Es sollte dann entsprechend das Wasservolumen, mit dem der Solebehälter bei der Druckmessung befüllt ist, zumindest im Wesentlichen dem gemessenen Wasservolumen V_{Wasser(gemessen)} entsprechen, also insbesondere nicht mehr als 10% oder nicht mehr als 5% von diesem abweichen.

Die Gleichung für P_{Wasserdruck} entspricht abgesehen davon, dass das gemessenen Wasservolumen V_{Wasser(gemessen)} eingesetzt wird, der Gleichung für den theoretischen Wasserdruck. Die obigen Erklärungen zum theoretischen Wasserdruck (vgl. A.1.3.1.a)), insbesondere bzgl. der Grundfläche, der Dichte und der Temperatur, sowie die Erklärungen zur Hinterlegung von Werten in der Auswerteinheit gelten entsprechend auch für den Wasserdruck P_{Wasserdruck} und sind hier anwendbar.

Bzgl. der Hinterlegung von Werten in der Auswerteinheit ist bei der Verwendung des mittels eines gemessenen Volumens bestimmten Wasserdruck P_{Wasserdruck} insbesondere eine Hinterlegung von Werten für ρWasser, A_{Solebehälter}, und g interessant. Es können zusätzlich natürlich auch Werte für P_{Wasserdruck (theoretisch)}) und/oder V_{Wasser} hinterlegt werden. Hierdurch wird vorteilhaft die Berechnung des Wasserverdrängungsdrucks in der Auswerteinheit wahlweise mittels des Wasserdrucks P_{Wasserdruck} oder des theoretischen Wasserdrucks vereinfacht.

A.1.3.2. Die weitere Verarbeitung des mittels Drucksensor gemessenen Wasserdrucks P_{Drucksensor} Der mittels Drucksensor gemessene Wert für den Wasserdruck P_{Drucksensor} kann nun, insbesondere unter Verwendung des oben diskutierten theoretischen Wasserdrucks oder des oben diskutierten mittels eines gemessenen Volumens bestimmten Wasserdrucks, weiterverarbeitet werden, bevorzugt in der Auswerteinheit.

Die Verarbeitung kann die Ermittlung beziehungsweise Berechnung eines für die Überwachung der Salzfüllung geeigneten Wertes umfassen, also insbesondere eines Wertes, der eine Aussage über die im Behälter enthaltene Salzmenge, die Zahl der noch möglichen Regenerationen und/oder einen Salzverbrauch während einer Regeneration ermöglicht.

Es können insbesondere der Wasserverdrängungsdruck P_{Wasserverdrängung} und/oder die Salzmasse m_{Salz} im Behälter bestimmt werden.

### a) Ermittlung des Wasserverdrängungsdrucks P_{Wasserverdrängung}

Gemäß einer Ausführungsform wird der gemessene Wasserdruck verwendet, um den Wasserverdrängungsdruck P_{Wasserverdrängung} zu berechnen. Diese Berechnung erfolgt üblicherweise in der Auswerteeinheit. Es sind mehrere Ausgestaltungsformen möglich.

Gemäß einer Ausgestaltungsform (A) wird der Wasserverdrängungsdruck mittels der Gleichung ${p}_{Wasserverdr ä ngung} \left[\frac{kg}{{s}^{2} ∗ m}\right] = {p}_{Drucksensor} \left[\frac{kg}{{s}^{2} ∗ m}\right] - {p}_{wasserdruck \left(theoretisch\right)} \left[\frac{kg}{{s}^{2} ∗ m}\right]$ bestimmt. Die obigen Erklärungen (vgl. insbesondere A.1.3.1. a)) zum theoretischen Wasserdruck P_{Wasserdruck(theoretisch)} und der Gleichung zu dessen Berechnung sind hierbei anwendbar.

Gemäß einer weiteren Ausgestaltungsform (B) wird das in den Solebehälter eingefüllte Wasservolumen gemessen und bevorzugt an die Auswerteeinheit übermittelt. Der übermittelte Wert wird dann auch bei der weiteren Berechnung des Wasserverdrängungsdrucks angesetzt. Die Ausgestaltungsform (B) kann wie folgt verwirklicht werden:
An einem Wasserzulauf des Solebehälters befindet sich ein Volumenstromsensor, der bei der Befüllung des Solebehälters mit Wasser das zugeführte Wasservolumen misst und zur Ermittlung des Wasserdrucks P_{Wasserdruck} an die Auswerteeinheit übermittelt, und der Wasserverdrängungsdruck P_{Wasserverdrängung} wird ermittelt mit der Gleichung ${p}_{Wasserverdr ä ngung} \left[\frac{kg}{{s}^{2} ∗ m}\right] = {p}_{Drucksensor} \left[\frac{kg}{{s}^{2} ∗ m}\right] - {p}_{wasserdruck} \left[\frac{kg}{{s}^{2} ∗ m}\right] .$

Die obigen Erklärungen (vgl. insbesondere A.1.3.1. b)) zum mittels Messung bestimmten Wasserdruck P_{Wasserdruck} und der Gleichung zu dessen Berechnung sind hierbei anwendbar.

Der nach Ausgestaltungsform (A) oder (B) ermittelte Wasserverdrängungsdruck P_{Wasserverdrängung} kann dann, bevorzugt in der Auswerteinheit, mit einem Druckschwellenwert für den Wasserverdrängungsdruck P_{Wasserverdrängung} verglichen werden. Der Druckschwellenwert kann in der Auswerteinheit hinterlegt sein. Ist der ermittelte Wasserverdrängungsdruck gleich dem oder kleiner als der Druckschwellenwert, kann durch die Auswerteeinheit oder einen Signalgeber, mit dem die Auswerteeinheit kommuniziert, ein Signal ausgelöst werden.
- Der Druckschwellenwert kann beispielsweise für eine Mindestsalzfüllung des Solebehälter repräsentativ sein.
- Der Druckschwellenwert kann dem Druck entsprechen, den ein Einfüllwasservolumen in Gegenwart einer Salzmasse im Solebehälter aufweist, die für eine vorbestimmte Anzahl an Regenerationen des Ionenaustauscher-Materials des Enthärters einer Vorbehandlungseinheit ausreicht, wobei die Anzahl der Regenerationen bevorzugt ausgewählt ist aus eine, zwei oder drei Regenerationen, am meisten bevorzugt eine Regeneration.

Der Nutzer kann so beispielsweise unmittelbar und in einfacher Weise informiert werden, dass Salz im Behälter nachgefüllt werden muss, und eine Schädigung oder ein Ausfall des Enthärters durch unzureichende Regeneration kann verhindert werden.

Es können auch mehrere Druckschwellenwerte berücksichtigt werden, insbesondere in der Auswerteeinheit hinterlegt sein. Die Signale können dann zur Unterscheidung der verschiedenen Druckschwellenwerte durch den Nutzer auch unterschiedlich ausgestaltet sein (beispielsweise gelbes Signallicht, wenn noch eine gewünschte Zahl an Regenerationen möglich ist, rotes Signallicht und/oder akustisches Signal, wenn eine Mindestsalzfüllung erreicht oder unterschritten wird, die nicht mehr für eine Regeneration ausreicht).

Das Auslösen des Signals kann selbsttätig erfolgen, wenn der ermittelte Wasserverdrängungsdruck gleich dem oder kleiner als der Druckschwellenwert ist. Das Signal kann ausgewählt sein aus einem akustischen Signal, einem optischen Signal, einem als Alarm ausgestalten Signal, einer Salznachfüllaufforderung, oder Kombinationen davon.

Zur Auslösung des Signals kann die Auswerteeinheit mit einem Signalgeber kommunizieren.

Zusätzlich oder alternativ kann ein Absperrorgan, bevorzugt ein Magnetventil, von der Auswerteeinheit oder dem Signalgeber angesteuert werden, um den Wasserzulauf von der Vorbehandlungseinheit in Richtung der Umkehrosmoseeinheit abzuschneiden. So kann verhindert werden, dass die Umkehrosmoseeinheit mit auf Grund mangelnder Regeneration des Ionenaustauscher-Materials unzureichend vorbehandeltem Wasser in Kontakt kommt. Hierdurch können insbesondere weiterreichende Beeinträchtigungen im Betriebsablauf und Schäden an der Umkehrosmoseeinheit beziehungsweise der Membran vermieden werden. Das Magnetventil kann gegebenenfalls auch direkt von der Vorbehandlungseinheit angesteuert werden.

### b) Ermittlung der Salzmasse m_{Salz}

Der gemessene Wasserdruck kann auch verwendet werden, um die im Solebehälter befindlichen Salzmasse m_{Salz} zu berechnen. Diese Berechnung erfolgt üblicherweise in der Auswerteeinheit. Die Ausführungsform kann mit der Berechnung des Wasserverdrängungsdrucks P_{Wasserverdrängung} und gegebenenfalls dessen Vergleich mit einem Druckschwellenwert kombiniert werden. Auch bei der Ermittlung der im Solebehälter befindlichen Salzmasse m_{Salz} sind mehrere Ausgestaltungsformen möglich. Es gilt insbesondere: ${V}_{Wasserverdr ä ngung} \left[{m}^{3}\right] = \frac{{p}_{Wasserverdr ä ngung} \left[\frac{kg}{{s}^{2} ∗ m}\right] ∗ {A}_{Solebeh ä lter} \left[{m}^{2}\right]}{{ρ}_{Wasser} \left[\frac{kg}{{m}^{3}}\right] ∗ g \left[\frac{m}{{s}^{2}}\right]}$ ${V}_{Salz} \left[{m}^{3}\right] = {V}_{Wasserverdr ä ngung} \left[{m}^{3}\right]$ ${m}_{Salz} \left[kg\right] = {ρ}_{Salz} \left[\frac{kg}{{m}^{3}}\right] ∗ {V}_{Salz} \left[{m}^{3}\right]$

Daraus folgt bei Verwendung des theoretischen Wasserdrucks P_{Wasserdruck (theoretisch)}: ${m}_{Salz} = {ρ}_{Salz} ∗ \frac{\left({p}_{Drucksensor}-{p}_{wasserdruck \left(theoretisch\right)}\right) ∗ {A}_{Solebeh ä lter}}{{ρ}_{Wasser} ∗ g}$

Ebenso folgt daraus bei Verwendung des mittels eines gemessenen Volumens bestimmten Wasserdrucks P_{Wasserdruck}: ${m}_{Salz} = {ρ}_{Salz} ∗ \frac{\left({p}_{Drucksensor}-{p}_{wasserdruck}\right) ∗ {A}_{Solebeh ä lter}}{{ρ}_{Wasser} ∗ g}$

Für den theoretischen Wasserdruck P_{Wasserdruck(theoretisch)}, den Wasserdruck P_{Wasserdruck}, die Grundfläche A_{Solebehälter}, g, und die Dichte der wässrigen Flüssigkeit ρ_{Wasser} sind dabei die obigen Erklärungen (vgl. insbesondere A.1.3.1. a), b)) anwendbar. Die Dichte des Salzes ρ_{Salz} ist die Dichte des im Solebehälter befindlichen Salzes und dem Fachmann bekannt. Die Dichte für Kochsalz oder NaCl kann mit etwa 2160 kg/m³, oder genauer 2161,5 kg/m³ angesetzt werden.

Gemäß einer Ausgestaltungsform (A') wird die zum Messzeitpunkt im Solebehälter befindliche Salzmasse m_{Salz} berechnet mittels der Gleichung ${m}_{Salz} = {ρ}_{Salz} . ∗ \frac{\left({p}_{Drucksensor}-{p}_{wasserdruck \left(theoretisch\right)}\right) ∗ {A}_{Solebehälter}}{{ρ}_{Wasser} ∗ g} ,$

Gemäß einer Ausgestaltungsform (B') befindet sich an einem Wasserzulauf des Solebehälters ein Volumenstromsensor, der bei der Befüllung des Solebehälters mit Wasser das zugeführte Wasservolumen misst und zur Ermittlung des Wasserdrucks P_{Wasserdruck} an die Auswerteeinheit übermittelt, und die im Solebehälter befindlichen Salzmasse m_{Salz} wird berechnet mittels der Gleichung ${m}_{Salz} = {ρ}_{Salz} ∗ \frac{\left({p}_{Drucksensor}-{p}_{wasserdruck}\right) ∗ {A}_{Solebehälter}}{{ρ}_{Wasser} ∗ g} .$

Basierend auf der ermittelte Salzmasse m_{Salz} sind weitere optionale Verfahrensschritte möglich.
- Insbesondere kann mittels der Salzmasse die Anzahl der Regenerationen, die mit der im Solebehälter befindlichen Salzmasse noch möglich sind, bestimmt werden.
- Zusätzlich oder alternativ kann mittels der Salzmasse die nachzufüllenden Salzmasse bestimmt werden.
- Weiter zusätzlich oder alternativ zu den vorgenannten auf der ermittelten Salzmasse basierenden Schritten ist auch der Vergleich, bevorzugt in der Auswerteinheit, der ermittelten Salzmasse mit einem, bevorzugt in der Auswerteeinheit hinterlegten, Salzmassenschwellenwert, möglich. Ist die ermittelte Salzmasse gleich dem oder kleiner als der Salzmassenschwellenwert, kann durch die Auswerteeinheit oder einen Signalgeber, mit dem die Auswerteeinheit kommuniziert, ein Signal ausgelöst werden. Die in der vorbeschriebenen Ausführungsform zum Druckschwellenwert dargestellten Vorteile treffen analog auch hier zu.
   ∘ Der Salzmassenschwellenwert kann für eine Mindestsalzmasse in dem Solebehälter repräsentativ sein.
   ∘ Der Salzmassenschwellenwert kann einer Salzmasse im Solebehälter entsprechen, die für eine vorbestimmte Anzahl an Regenerationen eines Ionenaustauscher-Materials eines Enthärters einer Vorbehandlungseinheit ausreicht, wobei die Anzahl bevorzugt ausgewählt ist aus eine, zwei oder drei Regenerationen, am meisten bevorzugt eine Regeneration.
   ∘ Es können auch mehrere Salzmassenschwellenwerte (bspw. gemäß den beiden vorgenannten Ausführungsformen) berücksichtigt werden, insbesondere in der Auswerteeinheit hinterlegt sein. Die Signale können dann zur Unterscheidung der verschiedenen Salzmassenschwellenwerte durch den Nutzer auch unterschiedlich ausgestaltet sein (etwa entsprechend dem für den Druckschwellenwert beschrieben Beispiel).
   ∘ das Auslösen des Signals kann selbsttätig erfolgen, wenn die ermittelte Salzmasse gleich dem oder kleiner als der Salzmassenschwellenwert ist und/oder das Signal kann ausgewählt sein aus einem akustischen Signal, einem optischen Signal, einem als Alarm ausgestalteten Signal, einer Salznachfüllaufforderung, oder Kombinationen davon, und/oder
   o Zusätzlich oder alternativ kann ein Absperrorgan, bevorzugt ein Magnetventil, von der Auswerteeinheit oder dem Signalgeber angesteuert werden, um den Wasserzulauf von der Vorbehandlungseinheit in Richtung der Umkehrosmoseeinheit abzuschneiden, mit den oben im Zusammenhang mit dem Druckschwellenwert beschriebenen Vorteilen.

### c) Abgleich des Wasserdrucks P_{Drucksensor} mit Druckschwellenwert für den Wasserdruck P_{Drucksensor}

Falls gewünscht, kann der gemessene Wasserdruck zur Weiterverarbeitung mit einem Druckschwellenwert für den Wasserdruck abgeglichen werden, was eine binäre Aussage (bspw. nächste Regeneration noch möglich [Ja/Nein]) erlaubt. Dies ist eine weitere, hinsichtlich ihrer Einfachheit vorteilhafte Ausführungsform, die natürlich auch mit einer oder mehreren der weitere hierin beschriebenen Ausführungsformen kombiniert werden kann, bei denen beispielweise der theoretische oder mittels eines gemessenen Volumens bestimmte Wasserdruck zum Einsatz kommt. Der Druckschwellenwert für den Wasserdruck P_{Drucksensor} kann dabei in der Auswerteinheit hinterlegt sein. Ist der gemessene Wert für den Wasserdruck P_{Drucksensor} gleich dem oder kleiner als der Druckschwellenwert, kann durch die Auswerteeinheit oder einen Signalgeber, mit dem die Auswerteeinheit kommuniziert, ein Signal ausgelöst werden, analog zur Offenbarung oben für den Druckschwellenwert des Wasserverdrängungsdruck P_{Wasserverdrängung}.

Der Drucksensor kann als Druckschalter dienen, beispielsweise, um ab wenigstens einem vordefinierten Schwellwert ein Signal auszugeben, beispielsweise bei Erreichen oder Unterschreiten eines Druckschwellenwertes.

### A.1.4. Kommunikation

Elemente des erfindungsgemäßen Systems (beispielsweise der Sensor und die Auswerteeinheit) können über Kommunikationsmittel kommunizieren, wie bereits vorstehend beschrieben. Dabei können Kommunikationsmittel zum Einsatz kommen wie auch unten für das System beschrieben, vgl. insbesondere B.1.6.

Auch kann eine Kommunikation mit mindestens einem weiteren Gerät erfolgen. Beispielsweise kann die Auswerteinheit mit mindestens einem nachgelagerten Gerät, insbesondere einer Umkehrosmoseeinheit, über ein Kommunikationsmittel kommunizieren. Dabei ist das Kommunikationsmittel (110) bevorzugt als Mittel zur digitalen Kommunikation (110), beispielsweise als Datenübertragungssystem (1101) oder Buskommunikation (1102), ausgebildet. "Nachgelagert" kann insbesondere als "der Vorbehandlungseinheit im Anlagenbetrieb nachgeordnet" oder "mit Weichwasser aus der Vorbehandlung versorgt" verstanden werden.

Die Kommunikation kann eine oder mehrere der folgenden Informationen, beispielsweise an eine Umkehrosmoseanlage oder einer Umkehrosmoseeinheit der Umkehrosmoseanlage übermitteln:
∘ Status des Regenerationsprozesses, insbesondere "Regenerationsprozess aktiv" und/oder "Regenerationsprozess nicht erfolgreich",
∘ Vorhandene Salzmasse im Solebehälter zu Beginn der Regeneration, und/oder
∘ Anzahl der verbleibenden Regenerationszyklen bis Salz im Solebehälter aufgebraucht, insbesondere "unzureichende Anzahl verbleibender Regenerationszyklen" bis Salz im Solebehälter aufgebraucht.

Die Umkehrosmoseanlage bzw. die Umkehrosmoseeinheit kann daraufhin eine oder mehrere Aktionen starten, insbesondere Aktionen ausgewählt aus Hartwassernotbetrieb, Anlagenstart bei aktiver Regeneration verschieben und/oder Anzeigen von Daten.

### A.1.5. Weitere Ausgestaltungen des Verfahrens

Typischerweise und bevorzugt ist der Solebehälter beim Messen des mindestens einen Messwertes, hier des Wasserdrucks P_{Drucksensor}, mit einem Wasservolumen und gegebenenfalls Salz, bevorzugt NaCl oder Kochsalz, befüllt. Das Wasservolumen kann als Reinwasser (insbesondere kurz nach der Befüllung) oder als Salzlösung, insbesondere als gesättigte Salzlösung vorliegen.

Gemäß einer bevorzugten Ausführungsform enthält der Solebehälter bei einer Befüllung mit einem Wasservolumen, insbesondere einem Einfüllwasservolumen, im Wesentlichen keine oder keine Flüssigkeit. Der Wasserdruck P_{Drucksensor} wird dann bevorzugt in dem mit dem Wasservolumen, insbesondere dem Einfüllwasservolumen, befüllten Solebehälter gemessen.

### A.1.5.1. Umgang mit Restflüssigkeitsvolumen

Gemäß einer weiteren Ausführungsform enthält der Solebehälter vor dem Befüllen mit einem Wasservolumen bereits ein Restflüssigkeitsvolumen (insbesondere ein Volumen von mehr als 10%, oder mehr als 5% des eingefüllten Wasservolumens, oder auch ein Restvolumen an Flüssigkeit ≥ 5 L, ≥ 10 L ≥ 15 L oder ≥ 20 L).

### Wie in diesem Fall beim Ansetzen der Dichte der wässrigen Flüssigkeit ρ_{Wasser} vorgegangen werden kann, wurde bereits oben beschrieben, vgl. A.1.3.1. a).

Es ist dann auch zweckmäßig, folgendes zu beachten:
- Wird beim Befüllen des Behälters das Wasservolumen mittels Volumenstromsensor erfasst, und vor der Befüllung liegt bereits ein Restwasservolumen im Behälter vor, sollte bei einer Berechnung von P_{Wasserdruck} nicht V_{Wasser(gemessen)}, sondern bevorzugt ein um das Restwasservolumen nach oben korrigierter Wasservolumenwert angesetzt werden (V_{Wasser(gemessen)} + Restwasservolumen).
- Analog kann statt eines festgelegten Wasservolumenwerts V_{Wasser} ein nach oben korrigierter Wert (Restwasservolumen + Wasservolumen, mit dem der Behälter befüllt wird) angesetzt werden.

Dabei können das Restwasservolumen und gegebenenfalls das Wasservolumen, mit dem der Behälter befüllt wird (sofern nicht schon über einen Volumenstromsensor gemessen) auf fachübliche Art bestimmt werden.

Eine vereinfachte und daher bevorzugte Vorgehensweise zum Umgang mit Restwasservolumina ist es, einen ein Restvolumen enthaltender Behälter so zu befüllen, dass insgesamt das Einfüllwasservolumen im Behälter vorliegt. Vereinfacht gesagt kann der Behälter so aufgefüllt werden, dass er wie vor einer Regeneration üblich befüllt ist. Es kann dann das Einfüllwasservolumen angesetzt werden, um den vom gemessenen Druck abzuziehenden erwarteten Druck zu berechnen. Es ist hierbei besonders vorteilhaft, die Druckmessung auch erst nach einer längeren Standzeit vorzunehmen (beispielsweise > 2h), weil dann die Dichte einer gesättigten Salzlösung als Dichte der wässrigen Flüssigkeit (ρ_{Wasser}) angesetzt werden kann. Die Druckmessung erfolgt dann im entsprechend mit dem Einfüllvolumen befüllten Behälter.

### A.1.5.2. Anordnung des Drucksensors bei der Messung

Der Drucksensor kann bei der Messung des Wasserdrucks P_{Drucksensor} wie unten für das System beschrieben (vgl. insbesondere B.1.1) am Solebehälter angeordnet sein. Ist der Drucksensor oberhalb eines Unterbodens des Solebehälters angeordnet, wird das Wasservolumen zwischen Unterboden und dem darüber platzierten Drucksensor nicht von der Druckmessung erfasst. Zur Kompensation kann da zur Berechnung des theoretischen Wasserdrucks P_{Wasser(theoretisch)} herangezogene Volumen V_{Wasser} durch Subtraktion des von der Druckmessung nicht erfassten Wasservolumens angepasst, sprich vermindert, werden. Analoges gilt bei der Berechnung des mittels des gemessenen Volumens bestimmten Wasserdrucks P_{Wasserdruck.}

### A.1.5.3. Weitere Verfahrensschritte

Das erfindungsgemäße Verfahren kann neben den vorstehend offenbarten Schritten auch ein oder mehrere weitere Schritte umfassen, insbesondere einen oder mehrere der folgenden Schritte:
- Befüllen des Solebehälters mit einem Wasservolumen, bevorzugt einem Einfüllwasservolumen, wobei optional
   ∘ der Solebehälter mindestens 1 h, mindestens 1,5 h, oder mindestens 2 h vor einer Regeneration mit dem Wasservolumen befüllt wird, um im Solebehälter enthaltenes Salz zu lösen,
   ∘ der Solebehälter im Anschluss an eine Regeneration mit dem Wasservolumen befüllt wird, bevorzugt innerhalb von 2 h, 1,5 h, 1 h oder 0,5 h nach einer Regeneration,
   o das Befüllen mit dem Wasservolumen von einer Vorbehandlungseinheit der Umkehrosmoseanlage verwaltet wird, bevorzugt von einem Steuerkopf der Vorbehandlungseinheit,
   ∘ der Solebehälter mit 20 L - 70 L, 30 L - 60 L, 35 L - 50 L oder etwa 40 L Wasser befüllt wird, und/oder
   ∘ das Wasser eine Temperatur ausgewählt aus 10 °C - 30 °C oder 12 °C - 25 °C hat.
- Befüllen des Solebehälters mit Salz, wobei optional
   ∘ der Solebehälter mit 8 kg - 40 kg, 20 kg - 30 kg, oder etwa 25 kg Salz, insbesondere Kochsalz oder NaCl, befüllt wird und/oder das Salz in Tablettenform vorliegt, und/oder
   ∘ der Solebehälter vor einer Regeneration mit Salz befüllt wird,
   ∘ der Solebehälter nach einer Regeneration mit Salz befüllt wird.
- Durchführen einer Regeneration einer Vorbehandlungseinheit der Umkehrosmoseanlage.

### A.2. Messung mit Füllstandsensor

Im Nachfolgenden wird auf eine weitere Ausführungsform eingegangen, in der ein Füllstandssensor zum Einsatz kommt. Dies kann zusätzlich oder alternativ zu den vorbeschriebenen, insbesondere druckmessungsbasierten, Ausführungsformen erfolgen. Die Ausführungsform ist frei mit einer oder mehreren der vorbeschriebenen Ausführungsformen kombinierbar. Hierdurch kann mit geringem apparativem Aufwand das oben beschriebene druckmessungsbasierte Verfahren weiter ausgestaltet werden, wobei dem Füllstandssensor dann bspw. die Funktion einer zusätzlichen Kontrolle eines Mindestsalzfüllstandes zukommen kann, was die Sicherheit und Robustheit des erfindungsgemäßen Verfahrens weiter erhöht.

In dieser Ausführungsform umfasst das erfindungsgemäße Verfahren zur Überwachung einer Salzfüllung eines Solebehälters einer Umkehrosmoseanlage:
- Messen eines Wasserfüllstandwerts einer wässrigen Flüssigkeit im Solebehälter mittels eines Füllstandssensors,
- optional Übermitteln des Wasserfüllstandwerts an mindestens eine Auswerteeinheit, und
- Verarbeiten des Wasserfüllstandwerts, optional in der mindestens einen Auswerteeinheit.

Die Positionierung des wenigstens einen Auslösefüllstandes und somit Auslöselevels kann der Höhe entsprechen, die ein Wasservolumen, insbesondere ein bekanntes Einfüllwasservolumen, im Solebehälter durch Volumenverdrängung des Salzes nur dann erreicht, wenn noch eine Mindestmenge an Salz im Behälter vorhanden ist, beispielsweise genügend Salz für wenigstens einen Regenerationsprozess.

Die Positionierung des Auslösefüllstandes im Solebehälter kann entsprechend für eine Mindestsalzfüllung des Solebehälter repräsentativ sein.

Insbesondere kann die Positionierung des Auslösefüllstandes im Solebehälter dem Wasserfüllstand im Solebehälter entsprechen, die ein Einfüllwasservolumen in Gegenwart einer Salzmasse im Solebehälter aufweist, die für eine vorbestimmte Anzahl an Regenerationen eines Ionenaustauscher-Materials eines Enthärters einer Vorbehandlungseinheit der Umkehrosmoseanlage ausreicht, wobei die Anzahl bevorzugt ausgewählt ist aus eine, zwei oder drei Regenerationen, am meisten bevorzugt eine Regeneration.

Wenn der bestimmte Wasserfüllstand unterhalb eines Auslösefüllstandes liegt oder diesem entspricht, kann ein Signal ausgelöst werden.

Das Auslösen des Signals kann selbsttätig erfolgen. Das Signal kann ausgewählt sein aus einem akustischen Signal, einem optischen Signal, einem als Alarm ausgestalteten Signal, einer Salznachfüllaufforderung, oder Kombinationen davon.

Auch kann in einer Ausführungsform die Auswerteeinheit oder das Signal ein Absperrorgan, bevorzugt ein Magnetventil, ansteuerten, um den Wasserzulauf von der Vorbehandlungseinheit in Richtung der Umkehrosmoseeinheit abzuschneiden.

Die obige Beschreibung des Verfahrens ist auch für die Ausführungsform, in der ein Füllstandssensor als einziger Sensor oder in Kombination mit weiteren Sensoren zum Einsatz kommt, anwendbar sofern sich nicht aus dem Kontext etwas anderes ergibt.

### B. System

In einem zweiten Aspekt wird auch eine Vorrichtung, nämlich ein System, zur Überwachung einer Salzfüllung eines Solebehälters für eine Umkehrosmoseanlage bereitgestellt. Das System kann im erfindungsgemäßen Verfahren eingesetzt und somit verwendet werden.

Das System umfasst mindestens einen Sensor zum Messen mindestens eines Messwertes mindestens einer Messgröße einer gegebenenfalls im Solebehälter enthaltenen wässrigen Flüssigkeit, wobei der mindestens einen Sensor zum Messen des mindestens eines Messwertes am oder im Solebehälter angeordnet ist, und wobei optional das System weiter eine Auswerteeinheit und/oder den Solebehälter umfasst.

Das System kann mindestens eine der folgenden Eigenschaften aufweisen:
(i) das System (120) umfasst einen Drucksensor (1011) und/oder oder ein Füllstandsensor (1012),
(ii) das System (120) umfasst einen Drucksensor (1011), der wenigstens in einem Teil, der mit der wässrigen Flüssigkeit in Kontakt gelangt, korrosionsresistent ist und/oder der eine bündig mit dem Drucksensorgehäuse abschließende Messmembran hat,
(iii) der mindestens eine Sensor (101), insbesondere Drucksensor (1011), ist am oder im Solebehälter (100) angebracht,
(iv) der mindestens eine Sensor (101), insbesondere Drucksensor (1011), ist mit dem Solebehälter (100) reversibel oder dauerhaft verbunden,
(v) der mindestens eine Sensor (101), insbesondere Drucksensor (1011), ist als integraler Bestandteil des Solebehälters (100) ausgebildet;
(vi) der mindestens eine Sensor (101), insbesondere Drucksensor (1011), ist mit der Auswerteeinheit (102) zur Übermittlung des Messwertes über ein Kommunikationsmittel (110) verbindbar oder verbunden, wobei das Kommunikationsmittel (110) bevorzugt als Mittel zur digitalen Kommunikation (110), beispielsweise als Datenübertragungssystem (1101) oder Buskommunikation (1102), ausgebildet ist.

Das System kann vorteilhaft und bevorzugt teil- oder vollautomatisch betreibbar sein. Es ist zur Durchführung des erfindungsgemäßen Verfahrens geeignet.

Das System umfasst bevorzugt die Auswerteeinheit. Entsprechend kann das System umfassen:
mindestens einen Sensor zum Messen mindestens eines Messwertes mindestens einer Messgröße einer gegebenenfalls im Solebehälter enthaltenen wässrigen Flüssigkeit, wobei der mindestens einen Sensor zum Messen des mindestens eines Messwertes am oder im Solebehälter angeordnet ist, eine Auswerteeinheit, und optional den Solebehälter.

Bevorzugt umfasst das erfindungsgemäße System zur Überwachung einer Salzfüllung eines Solebehälters für eine Umkehrosmoseanlage mindestens einen Drucksensor zum Messen eines Messwertes des Wasserdrucks P_{Drucksensor} einer gegebenenfalls im Solebehälter enthaltenen wässrigen Flüssigkeit, wobei der Drucksensor zum Messen des Wasserdrucks P_{Drucksensor} der wässrigen Flüssigkeit am Solebehälter angeordnet ist, eine Auswerteinheit, in der Wasserdrucks P_{Drucksensor} verarbeitet werden kann, und optional den Solebehälter.

Es wird nun zunächst der optional im System umfasste Solebehälter näher beschrieben. Dies scheint zweckmäßig, zum einen, da es bevorzugt ist, dass das System den Solebehälter umfasst, und auch da im Folgenden, insbesondere bei der Beschreibung möglicher Positionierungen des Drucksensors, dann auf diese Eigenschaften verwiesen werden kann.

Solebehälter sind aus dem Stand der Technik bekannt, und es können entsprechend vorbekannte Solebehälter eingesetzt bzw. in das System integriert werden. Dafür kann der Solebehälter durch Anordnung des Sensors am oder im Solebehälter modifiziert werden.

Der Solebehälters kann zylindrisch oder im Wesentlichen zylindrisch sein.

Der Solebehälter kann einen Unterboden mit einer Grundfläche A_{Solebehälter} und mindestens eine sich daran anschließende Seitenwand aufweisen. Durch den Unterboden und die mindestens eine sich daran anschließende Seitenwand kann ein Innenraum des Solebehälters gebildet werden.

Der Solebehälter verfügt über eine oder mehrere Öffnungen für die Befüllung mit Salz und/oder Wasser und Salz und gegebenenfalls einen Deckel. Der Solebehälter kann für die Befüllung mit Wasser mit einem Wasserzulauf, beispielsweise einem Betriebswasserzulauf, und/oder zur Entnahme von Salzlösung oder Sole mit einer Soleleitung verbindbar oder verbunden sein.

Der Solebehälter kann mit einem ein Ionenaustauscher-Material umfassenden Enthärter einer Vorbehandlungseinheit zur Regeneration des Ionenaustauscher-Materials verbindbar oder verbunden sei. Er kann auch Teil der Vorbehandlungseinheit ausgestaltet sein.

Der Solebehälter kann einen wasserdurchlässigen Salzträger umfassen, beispielsweise ein Sieb, ein Netz oder einen Sack. Der Salzträger kann festes Salz, beispielsweise Salztabletten zurückhalten, ist aber hinreichend wasserdurchlässig, um die Bildung einer Salzlösung zu ermöglichen. Entsprechende Salzträger und geeignete Anordnungen im oder am Behälter sind dem Fachmann auch bekannt. Der Salzträger ist bevorzugt so angeordnet, dass das feste Salz über einer parallel zum Unterboden des Solebehälters befindlichen Ebene, welche eine Seitenwand des Solebehälters direkt oberhalb des Drucksensors schneidet, angeordnet ist.

Der Solebehälter kann Wasser, Salzlösung und/oder Salz, üblicherweise in Form von Salztabletten, enthalten. In der Regel wird Kochsalz oder NaCl eingesetzt. Die Volumina bzw. Mengen können dabei je nach Behältergröße und Betriebszustand variieren, der Fachmann ist mit üblichen und geeigneten Volumina bzw. Mengen vertraut.

In einer beispielhaften Ausführungsform kann der Behälter 8 kg - 40 kg, 20 kg - 30 kg, oder etwa 25 kg Salz, beispielsweise in Form von Salztabletten oder grobkörnigem Salz enthalten. Der Behälter kann mit einem Wasservolumen von 20 L - 70 L, 30 L - 60 L, 35 L - 50 L oder etwa 40 L befüllt sein. Er kann eine Salzlösung, insbesondere eine gesättigte Salzlösung enthalten.

Das System kann neben dem Sensor, insbesondere dem Drucksensor, und bevorzugt der Auswerteeinheit, auch weitere Komponenten umfassen, wie:
- eine Vorbehandlungseinheit für eine Umkehrosmoseeinheit, die Vorbehandlungseinheit umfassend mindestens einen ein Ionenaustauscher-Material umfassenden Enthärter, und
- den Solebehälter.

Vorteilhaft kann dabei die Vorbehandlungseinheit ausgestaltet sein, um die Befüllung des Solebehälters mit Wasser zu verwalten. Die Vorbehandlungseinheit kann hierzu einen Steuerkopf umfassen. Die Vorbehandlungseinheit kann mit der Umkehrosmoseeinheit zur Zufuhr von vorbehandeltem Wasser an die Umkehrosmoseeinheit verbindbar oder verbunden sein.

Der Solebehälter kann dabei eine oder mehrere der vorstehend für den Solebehälter offenbarten Eigenschaften aufweisen, insbesondere als Teil der Vorbehandlungseinheit ausgestaltet und/oder mit dem Enthärter zur Regeneration des Ionenaustauscher-Materials verbindbar oder verbunden sein.

Das System kann neben dem Sensor, insbesondere dem Drucksensor, und bevorzugt der Auswerteeinheit auch eine Umkehrosmoseeinheit umfassen. Diese umfasst zumindest eine Membran für die Umkehrosmose.

Folglich kann das System neben dem Sensor, insbesondere dem Drucksensor, und bevorzugt der Auswerteeinheit auch umfassen:
- eine Vorbehandlungseinheit für eine Umkehrosmoseeinheit, die Vorbehandlungseinheit umfassend mindestens einen ein Ionenaustauscher-Material umfassenden Enthärter,
- den Solebehälter,
- und eine Umkehrosmoseeinheit, die mit der Vorbehandlungseinheit zur Zufuhr von vorbehandeltem Wasser an die Umkehrosmoseeinheit verbindbar oder verbunden ist.

In vorteilhafter Weise kann mit dem erfindungsgemäßen System, insbesondere bei Verwendung eines Drucksensors, die Salzfüllung des Solebehälters beispielsweise auf mindestens 2,5 kg genau, mindestens 2 kg genau, mindestens 1 kg genau, oder mindestens 500 g genau bestimmt werden. Bevorzugt erfolgt die Bestimmung auf 1 kg bis 2 kg genau, oder auf 1 kg genau. Die im Solebehälter befindliche Salzmasse kann bspw. auf mindestens 10% genau oder mindestens 8%, mindestens 5% oder mindestens 2% genau bestimmt werden. Bevorzugt wird die Masse auf mindestens 5% genau bestimmt.

### B.1. System mit Drucksensor

Ein bevorzugtes System ermöglicht die druckbasierte Überwachung der Salzfüllung. Entsprechend wird ein System zur Überwachung einer Salzfüllung eines Solebehälters für eine Umkehrosmoseanlage bereitgestellt, dadurch gekennzeichnet, dass das System mindestens einen Drucksensor zum Messen eines Messwertes des Wasserdrucks P_{Drucksensor} einer gegebenenfalls im Solebehälter enthaltenen wässrigen Flüssigkeit umfasst, wobei der Drucksensor zum Messen des Wasserdrucks P_{Drucksensor} der wässrigen Flüssigkeit am Solebehälter angeordnet ist, und wobei das System optional aber bevorzugt weiter den Solebehälter umfasst. Auf die obigen Erklärungen (vgl. insbesondere B.) zum Solebehälter und möglicher Ausgestaltungen des Solebehälters wird Bezug genommen.

Das System kann umfassen:
- den mindestens einen Drucksensor zum Messen eines Messwertes des Wasserdrucks P_{Drucksensor} einer gegebenenfalls im Solebehälter enthaltenen wässrigen Flüssigkeit, wobei der Drucksensor zum Messen des Wasserdrucks P_{Drucksensor} der wässrigen Flüssigkeit am Solebehälter angeordnet ist,
- eine Auswerteinheit, in der Wasserdrucks P_{Drucksensor} verarbeitet werden kann, und
- den Solebehälter.

Die Auswerteeinheit unterstützt dabei in vorteilhafter Weise einen teil- oder vollautomatischen Betrieb des Systems. Das System kann weiter umfassen:
- eine Vorbehandlungseinheit für eine Umkehrosmoseeinheit, die Vorbehandlungseinheit umfassend mindestens einen ein Ionenaustauscher-Material umfassenden Enthärter, und
- optional eine Umkehrosmoseeinheit, die mit der Vorbehandlungseinheit zur Zufuhr von vorbehandeltem Wasser an die Umkehrosmoseeinheit verbindbar oder verbunden ist.

Zu möglichen Ausgestaltungen insbesondere der Vorbehandlungseinheit und des Solebehälters wird auf die obigen Ausführungen (vgl. insbesondere B.) verwiesen.

### B.1.1. Drucksensor

Der Drucksensor ist zum Messen eines Wasserdrucks P_{Drucksensor} der wässrigen Flüssigkeit am oder im Solebehälter angeordnet, insbesondere mit dem Solebehälter (100) reversibel oder dauerhaft verbunden oder als integraler Bestandteil des Solebehälters (100) ausgebildet.

In bevorzugter Weise wird der Relativdruck gemessen, da entsprechende Sensoren günstiger und einfacher in der Handhabung sind, und die nachfolgenden Erklärungen erwähnen entsprechend eine relative Druckmessung.

Eine absolute Druckmessung ist ebenfalls möglich, aber weniger bevorzugt als die Relativdruckmessung. Bei der Verwendung eines Absolutdrucksensors statt eines Relativdrucksensors muss entweder ein gemittelter ortsabhängiger atmosphärischer Druck oder ein gemittelter ortsunabhängiger Druck abgezogen werden, um den tatsächlichen Wasserdruck zu berechnen.

In der Praxis ist es bevorzugt, den Drucksensor in der Nähe des Unterbodens (1001) des Solebehälters an einer Seitenwand (1002) des Solebehälters (100) anzuordnen. Hierdurch ist die Eignung und somit Flexibilität hinsichtlich verschiedener Flüssigkeitsvolumina und Behältergeometrien besonders hoch.

Der Drucksensor kann in dieser bevorzugten Ausführungsform an einer Seitenwand des Solebehälters in der Nähe des Unterbodens, insbesondere bis zu 10 cm, bis zu 8 cm, bis zu 5 cm, bis zu 4 cm oder etwa 3 cm vom Unterboden des Solebehälters entfernt, angeordnet sein. Die Entfernung des Drucksensors vom Unterboden kann auf der Seitenwand gemessen werden. Sie kann der kürzesten Strecke zwischen Drucksensor (insbesondere dessen unterem, zum Unterboden hin orientierten Rand) und Unterboden entsprechen. Der Drucksensor kann an einer Seitenwand im unteren Fünftel, unteren Sechstel oder unteren Zehntel der Seitenwand oder der Behälterhöhe angeordnet sein. Das untere Fünftel ist dabei das Fünftel, das dem Unterboden am nächsten liegt. Die Behälterhöhe kann der vertikalen Distanz zwischen Unterboden und oberer Öffnung oder Oberboden eines Behälters entsprechen.

Die Anordnung des Drucksensors ist aber nicht auf die bevorzugte Anordnung in Nähe des Unterbodens des Solebehälters beschränkt.

Allgemeiner gefasst kann der Drucksensor auch so angeordnet sein, dass er bei relativer Druckmessung einen Druckmesswert > 0 Pa liefert, wenn der Solebehälter gefüllt ist, insbesondere mit einem Einfüllwasservolumen gefüllt ist, und eine für eine Regeneration benötigte Mindestmasse an Salz enthält, wobei der Druckmesswert 0 Pa ist, wenn die Mindestmasse an Salz unterschritten ist. Der Drucksensor kann folglich so angeordnet sein, dass erst durch die in der Gegenwart der für eine Regeneration benötigte Mindestmasse an Salz verursachte Volumenverdrängung der Flüssigkeitsspiegel im gefüllten Solebehälter hoch genug steht, dass der Drucksensor einen Flüssigkeitsdruck abnehmen kann und bei relativer Druckmessung einen Druckmesswert > 0 Pa liefert. Bei dieser Ausführungsform zeigt ein Druckmesswert von 0 Pa an, dass keine Regeneration möglich ist, da die minimale Salzmenge nicht vorhanden ist (und/oder bspw. der Behälter leer ist).

Hierbei kann in einer vorteilhaften Ausführungsform beispielsweise mittels eines externen Signals an die Auswerteeinheit zwischen einer zu geringen Salzmenge im Solebehälter und einem leeren Solebehälter unterschieden werden. Die Auswerteeinheit kann somit ein externes Signal zur Unterscheidung zwischen einer zu geringen Salzmenge im Solebehälter und einem leeren Solebehälter erhalten, bzw. es kann ein entsprechendes Signal an die Auswerteeinheit bereitgestellt werden, Die Ausführungsform mit externem Signal ist beispielsweise dann besonders vorteilhaft, wenn bei unzureichender Regeneration ein Magnetventil geschlossen und/oder ein Alarmsignal, bspw. über eine Datenleitung, ausgegeben werden soll. Das externe Signal kann ein Signal der Vorbehandlungseinheit, insbesondere eines Regenerationssteuerkopfs der Vorbehandlungseinheit, sein, das bspw. eine laufende Regeneration anzeigt.

Ebenso kann der Drucksensor so angeordnet sein, dass er bei relativer Druckmessung einen Druckmesswert > 0 Pa liefert, wenn der Solebehälter mit einem Einfüllwasservolumen gefüllt ist, der Behälter aber kein Salz enthält, wobei der Druckmesswert 0 Pa ist, wenn das Einfüllwasservolumen unterschritten ist. Der Drucksensor kann folglich so angeordnet sein, dass bereits ohne Salz der Flüssigkeitsspiegel, insbesondere der Flüssigkeitsspiegel eines Einfüllwasservolumens, im gefüllten Solebehälter hoch genug steht, dass der Drucksensor einen Flüssigkeitsdruck abnehmen kann und bei relativer Druckmessung einen Druckmesswert > 0 Pa liefert. Ein höherer Druck als für die Befüllung ausschließlich mit der Flüssigkeit ergibt sich dann in Abhängigkeit von der im Behälter enthaltenen Salzmenge aufgrund deren Volumenverdrängung. Ein Vorteil dieser Ausführung ist, dass sie die Unterscheidung zwischen bspw. einem leeren oder lediglich mit Salz befülltem Behälter und einem Behälter, der für den Regenerationsprozess vorbereitet wird, insbesondere bereits im Wesentlichen oder vollständig mit Flüssigkeit gefüllt ist, ermöglicht.

Das Einfüllwasservolumen entspricht dabei jeweils bevorzugt dem Wasservolumen, mit dem der Solebehälter zur Herstellung einer Salzlösung für die Regeneration befüllt wird, wenn er noch keine Flüssigkeit enthält.

Bei den drei vorbeschriebenen Ausführungsformen ist der Drucksensor oberhalb des Unterbodens angeordnet, wodurch das Wasservolumen zwischen Unterboden und dem darüber platzierten Drucksensor nicht von der Druckmessung erfasst wird. Dies kann zu einer Unterschätzung der tatsächlich im Solebehälter befindlichen Salzmenge führen. Es ist daher in der Regel zweckmäßig, zur Berechnung des theoretischen Wasserdrucks P_{Wasser(theoretisch)} herangezogene Volumen V_{Wasser} durch Subtraktion des von der Druckmessung nicht erfassten Wasservolumens anzupassen, sprich zu vermindern. Analoges gilt bei der Berechnung des mittels des gemessenen Volumens bestimmten Wasserdrucks P_{Wasserdruck.} Auch hier ist es in der Regel zweckmäßig, das Volumen V_{Wasser(gemessen)} durch Subtraktion des von der Druckmessung nicht erfassten Wasservolumens zu vermindern. Das Volumen kann dabei insbesondere über die Geometrie des Solebehälters ermittelt werden.

Ein Salzträger ist bei den drei vorbeschriebenen Ausführungsformen in Relation zum Drucksensor bevorzugt so im Solebehälter angeordnet, dass das feste Salz über einer parallel zum Unterboden des Solebehälters befindlichen Ebene, welche eine Seitenwand des Solebehälters direkt oberhalb des Drucksensors schneidet, angeordnet ist. Der Behälter kann über einen entsprechenden Zwischenboden und/oder ein Salznetz verfügen, welches oberhalb der Ebene angeordnet ist.

In einer weiteren Ausgestaltung ist der Drucksensor am Unterboden des Solebehälters angeordnet und die Druckmessung erfolgt von unten durch den Behälterboden. Hier ist bei der Druckmessung das komplette Wasservolumen erfassbar und eine Berechnungsanpassung nicht erforderlich. Weniger vorteilhaft an dieser Variante ist, dass Schwebstoffe, Ablagerungen und Verschmutzungen mit der Zeit zu einer Beeinträchtigung des Sensors führen können. Dem kann bis zu einem gewissen Grad dadurch entgegengewirkt werden, dass über dem Sensor behälterinnenseitig ein mechanischer Schutz, bspw. ein Gitter oder Netz vorgesehen wird, der hinreichend wasserdurchlässig ist und die Sensormessung nicht beeinträchtigt. Die Schutzfunktion kann auch von einem Salzträger vermittelt werden.

Da der Drucksensor mit Salz und Salzlösung in Kontakt kommt, ist der Sensor bevorzugt, zumindest im medienberührenden Teil, korrosionsresistent ausgebildet. Hierfür kann auf Sensoren zurückgegriffen werden, die wenigstens im medienberührenden Teil aus korrosionsresistenten Materialien (bspw. aus der Materialgruppe AISI 316L) gefertigt sind.

Es ist auch bevorzugt, dass der Drucksensor über eine außenliegende Messmembran verfügt. Ein Drucksensor, der eine bündig mit dem Drucksensorgehäuse abschließende Messmembran hat, ist vorteilhaft. Da die Messmembran den Sensor gegenüber der Flüssigkeit bündig abschließt, wird die Bildung von Ablagerungen und Verstopfung (insbesondere mit Salzkristallen) gegenüber Drucksensoren mit offenem Kanal vermieden oder vermindert.

### B.1.2. Auswerteeinheit

Gemäß einer bevorzugten, der Automatisierung dienlichen, Ausführungsform umfasst das System mindestens eine Auswerteeinheit, wobei optional
(i) die Auswerteinheit als Teil des Sensors ausgebildet ist,
(ii) die Auswerteinheit mit dem Sensor über ein Kommunikationsmittel verbindbar oder verbunden ist,
(iii) die Auswerteinheit der Verarbeitung des mindestens einen Messwertes dient,
(iv) die Auswerteinheit über einen Speicher, bevorzugt einen persistenten Speicher, verfügt, in dem ein oder mehrere Werte hinterlegt sind, beispielsweise:
   ∘ mindestens ein theoretischen Wasserdruck P _{Wasserdruck (theoretisch)},
   ∘ mindestens ein Wasservolumenwert V_{Wasser},
   ∘ einer Grundfläche A_{Solebehälter} des Solebehälters,
   ∘ ein Wert für die Erdbeschleunigung g,
   ∘ mindestens ein Wert für die Dichte einer wässrigen Flüssigkeit ρ_{Wasser},
   ∘ mindestens ein Temperaturwert zur Bestimmung oder Korrektur der Dichte einer wässrigen Flüssigkeit,
   ∘ mindestens ein Druckschwellenwert,
   ∘ mindestens ein Salzmassenschwellenwert,
   ∘ ein aufgrund einer Anordnung des Drucksensors oberhalb des Unterbodens nicht in eine Druckmessung einfließendes Volumen zur Korrektur eines Wasservolumenwertes V_{Wasser} und/oder V_{Wasser(gemessen)}, und/oder
   ∘ mindestens ein Flüssigkeitsstandschwellenwert, und/oder
(v) die Auswerteinheit (102) mit mindestens einem nachgelagerten Gerät, insbesondere einer Umkehrosmoseeinheit (107), über ein Kommunikationsmittel (110) verbindbar oder verbunden ist, wobei das Kommunikationsmittel (110) bevorzugt als Mittel zur digitalen Kommunikation (110), beispielsweise als Datenübertragungssystem (1101) oder Buskommunikation (1102), ausgebildet ist.

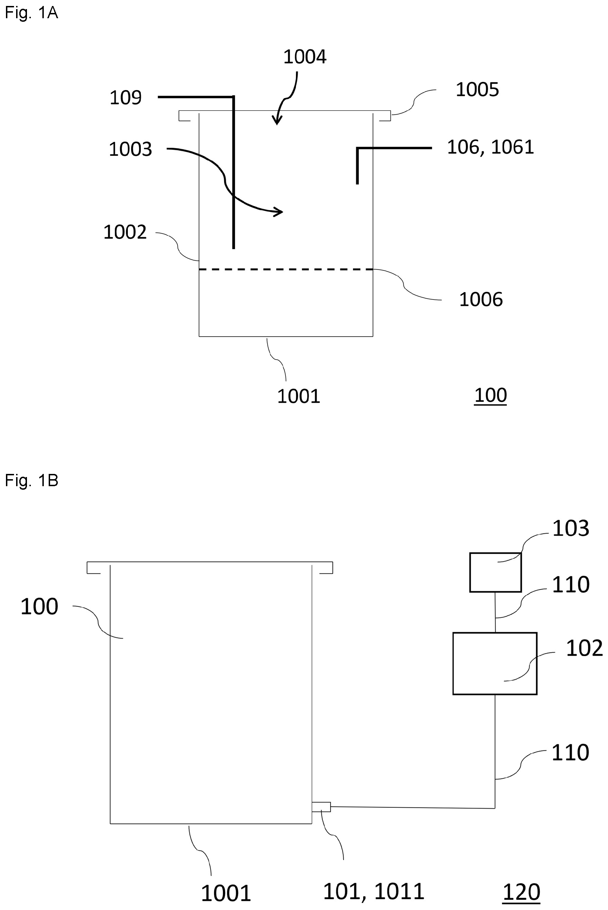

Fig. 1A zeigt schematisch einen beispielhaften, nicht beschränkenden Aufbau eines Solebehälters (100). Der Solebehälter (100) weist einen Unterboden (1001) und mindestens eine Seitenwand (1002) auf, die zusammen mit dem Unterboden (1001) den Behälterinnenraum (1003) definiert. Der Behälter verfügt über eine gegenüber des Unterbodens (1001) liegende Öffnung (1004), die in Fig. 1A von einem Deckel (1005) abgedeckt wird. Im Innenraum (1003) des Solebehälters (100) befindet sich ein wasserdurchlässiger Salzträger (1006), der als Träger für Salztabletten (nicht gezeigt) dient. Weiter gezeigt, aber hier nicht Teil des Solebehälters (100), ist eine Wasserleitung (106), genauer ein Wasserzulauf (1061), zur Befüllung des Solebehälters (100) mit Wasser. Der Wasserzulauf (1061) ragt über eine Öffnung an der Seitenwand (nicht gesondert gezeigt) in den Solebehälter hinein. Ebenfalls weiter gezeigt, aber hier nicht Teil des Solebehälters (100), ist eine Soleleitung (109) zur Entnahme einer gesättigten Salzlösung aus dem Solebehälter. Die Soleleitung ragt durch eine Öffnung (hier nicht gezeigt) im Deckel (1005) in den Innenraum (1003).

Fig. 1B zeigt schematisch einen beispielhaften, nicht beschränkenden Aufbau eines erfindungsgemäßen Systems (120) zur Überwachung einer Salzfüllung eines Solebehälters (100). Ein Sensor (101), hier als Drucksensor (1011) ausgestaltet, ist zum Messen des Drucks einer gegebenenfalls im Solebehälter (100) enthaltenen wässrigen Flüssigkeit (beispielsweise Wasser oder eine gesättigte Salzlösung, hier nicht gezeigt) am Solebehälter (100) angeordnet, hier entsprechend einer bevorzugten Ausführungsform in der Nähe des Unterbodens (1001) des Solebehälters (100). Der Drucksensor (1011) kann den Druck im Solebehälter (100) messen und ist mit einer Auswerteeinheit (102) verbunden, die in der hier gezeigten bevorzugten Ausführungsform ebenfalls Teil des Systems (120) ist. Der Solebehälter (100) ist hier ebenfalls Teil des Systems (120). Sobald ein Regenerationsprozess gestartet wird, wird der Solebehälter (100) mit Wasser gefüllt, um darin befindliche Salztabletten zu lösen. Der Befüllungsprozess kann von einer Vorbehandlungseinheit (hier nicht gezeigt), bspw. deren Steuerkopf, verwaltet werden. Die Geometrie des Solebehälter (100) sowie das Füllvolumen sind bekannt, und der theoretische Wasserdruck P_{Wasserdruck(theoretisch)} kann wie oben dargelegt berechnet werden und in der Auswerteeinheit (102) bzw. deren persistentem Speicher hinterlegt sein. Der tatsächliche Druck P_{Drucksensor} im Solebehälter (100) wird über den Drucksensor (101, 1001) gemessen und über ein Kommunikationsmittel (110) an die Auswerteeinheit (102) übermittelt. Nun können der Wasserverdrängungsdruck P_{Wasserverdrängung} und/oder die Salzmasse m_{Salz} berechnet werden, mit: ${p}_{Wasserverdrängung} \left[\frac{kg}{{s}^{2} ∗ m}\right] = {p}_{Drucksensor} \left[\frac{kg}{{s}^{2} ∗ m}\right] - {p}_{wasserdruck} \left[\frac{kg}{{s}^{2} ∗ m}\right]$ ${m}_{Salz} = {ρ}_{Salz} ∗ \frac{\left({p}_{Drucksensor}-{p}_{wasserdruck \left(theoretisch\right)}\right) ∗ {A}_{Solebeh ä lter}}{{ρ}_{Wasser} ∗ g}$

Auf die obigen Erläuterungen zu diesen Gleichungen bei der Diskussion des Verfahrens wird verwiesen. Alternativ zur Bestimmung des Salzgewichts bzw. der Salzmasse kann auch P_{Wasserverdrängung} mit einem Druckschwellenwert verglichen werden, um die Auswertung zu vereinfachen. Sobald das Salzgewicht unter einen Grenzwert fällt, kann die Auswerteeinheit (102) ein optisches und/oder akustisches Signal triggern. Gegebenenfalls kann die Auswerteeinheit (102) hierzu über ein Kommunikationsmittel (110) mit einem Signalgeber (103) kommunizieren, der dann das Signal abgibt. Der Signalgeber (103) ist optionaler Bestandteil der hier gezeigten Ausführungsform. Die Ausführungsform mit Signalgeber (103) wird nachfolgend weiter beschrieben.

### B.1.3. Signalgeber

Das System kann auch ein Signalgeber umfassen. Dieser kann als Teil der Auswerteinheit ausgebildet sein. Alternativ ist der Signalgeber ein eigenständiges Element des Systems. Bevorzugt ist dann die Auswerteinheit mit dem Signalgeber über ein Kommunikationsmittel verbindbar oder verbunden, wobei das Kommunikationsmittel (110) bevorzugt als Mittel zur digitalen Kommunikation (110), beispielsweise als Datenübertragungssystem (1101) oder Buskommunikation (1102), ausgebildet ist. Die im Zusammenhang mit dem Verfahren des ersten Aspekts bereitgestellte Offenbarung zum Signalgeber gilt entsprechend für das System.

### B.1.4. Volumenstromsensor

Gemäß einer Ausführungsform umfasst das System einen Volumenstromsensor zur Messung des bei der Befüllung des Solebehälters zugeführten Wasservolumens V_{Wasser(gemessen)}. Dies hat den Vorteil, dass die Berechnung des Wasserdrucks P_{Wasserdruck} anhand des gemessenen Wasservolumens erfolgen kann, wodurch eine höhere Genauigkeit und auch Flexibilität bezüglich des bei der Berechnung anzusetzenden Volumens erreicht werden kann. Typischerweise ist der Volumenstromsensor an einem Wasserzulauf des Solebehälters angeordnet.

Es wird auf die obigen Ausführungen zu Messung des Volumens und zu P_{Wasserdruck} (vgl. insbesondere in A.1.3.1. b)) Bezug genommen. Die Ausführungen sie sind hier entsprechend anwendbar.

Der Volumenstromsensor kann mit der Auswerteinheit über ein Kommunikationsmittel verbindbar oder verbunden sein, das wiederum bevorzugt als Mittel zur digitalen Kommunikation, beispielsweise als Datenübertragungssystem oder Buskommunikation, ausgebildet ist. Es kann insbesondere eine Übermittlung des Werts für das gemessene Wasservolumen V_{Wasser(gemessen)} an die Auswerteeinheit erfolgen, wo der Wert dann, wie oben bereits für das Verfahren dargelegt, weiterverarbeitet werden kann.

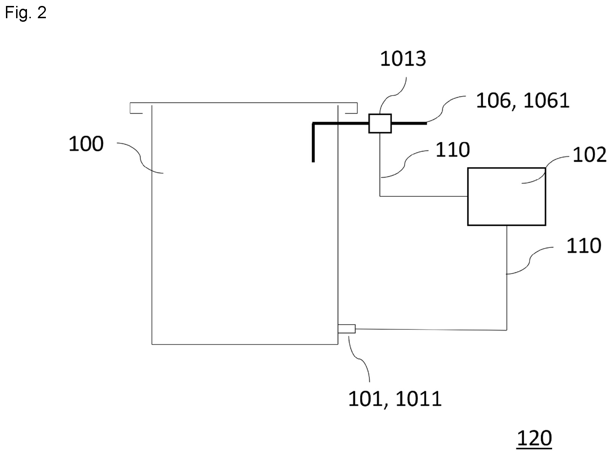

Fig. 2 zeigt schematisch einen beispielhaften, nicht beschränkenden Aufbau eines erfindungsgemäßen Systems (120) mit Volumenstromsensor (1013). Am Solebehälter (100) (gegebenenfalls Teil des Systems (120)) ist ein Drucksensor (101, 1011) angeordnet und mit der Auswerteeinheit (102) mittels eines Kommunikationsmittels (110) verbunden. Weiter befindet sich am Wasserzulauf (106, 1061) für den Solebehälter (100) ein Volumenstromsensor (1013). Der Wasserzulauf (106, 1061) selbst ist hier, im Gegensatz zum Volumenstromsensor (1013), nicht Teil des Systems (120). Der Volumenstromsensor (1013) ist mit der Auswerteeinheit (102) über ein Kommunikationsmittel (110) verbunden, und kann bei Befüllung des Solebehälters (100) mit Wasser das eingefüllte Volumen messen und an die Auswerteinheit (102) übermitteln. Die Berechnung des Wasserdrucks kann dann anhand des gemessenen Volumenstroms beziehungsweise Wasservolumens V_{Wasser(gemessen)} erfolgen, wie bereits oben dargelegt (vgl. insbesondere A.1.3.b)). ${p}_{wasserdruck} \left[\frac{kg}{{s}^{2} ∗ m}\right] = \frac{{ρ}_{Wasser} \left[\frac{kg}{{m}^{2}}\right] ∗ {V}_{wasser} \left(gemessen\right) \left[{m}^{3}\right] ∗ g \left[\frac{m}{{s}^{2}}\right]}{{A}_{Solebeh ä lter \left[{m}^{2}\right]}}$

### B.1.5. Absperrorgan

Gemäß einer Ausführungsform umfasst das System ein Absperrorgan, bevorzugt ein Magnetventil. Das Absperrorgan ist bevorzugt hinter der Vorbehandlungseinheit so an einer Wasserleitung angeordnet, dass es den Wasserzulauf von der Vorbehandlungseinheit zu dahinterliegenden Geräten, wie der Umkehrosmoseeinheit, abschneiden kann.

Das Absperrorgan bzw. das Magnetventil ist zur Unterbrechung einer Wasserzufuhr von der Vorbehandlungseinheit hin zu dahinterliegenden Geräten, insbesondere der Umkehrosmoseeinheit, ansteuerbar, wie auch oben für das Verfahren bereits dargelegt. Insbesondere kann das Absperrorgan von der Auswerteeinheit angesteuert werden und hierzu mit der Auswerteeinheit über ein Kommunikationsmittel verbindbar oder verbunden sein. Zusätzlich oder alternativ kann das Absperrorgan auch direkt von der Vorbehandlungseinheit angesteuert werden, und hierzu mit dieser über ein Kommunikationsmittel verbindbar oder verbunden sein. Ebenso denkbar ist eine Ansteuerung über einen Signalgeber, der seinerseits von der Auswerteeinheit angesteuert wird. Das Kommunikationsmittel ist dabei bevorzugt als Mittel zur digitalen Kommunikation, beispielsweise als Datenübertragungssystem oder Buskommunikation, ausgebildet.

Der Wasserzulauf von der Vorbehandlungseinheit in Richtung dahinterliegenden Geräte, insbesondere der Umkehrosmoseeinheit, kann so bei Bedarf abgeschnitten werden. Hierdurch können Verunreinigungen und Beschädigungen der Umkehrosmoseeinheit, insbesondere deren Membran, vermindert oder vermieden werden und die Betriebsfähigkeit der Umkehrosmoseeinheit erhalten bleiben. Es ist so auch möglich, die Weichwasserzufuhr von der Vorbehandlungseinheit an die Umkehrosmoseeinheit während eines Regenerationsprozesses zu unterbrechen, sollte dies aus beispielsweise regulatorischer Sicht von Vorteil sein.

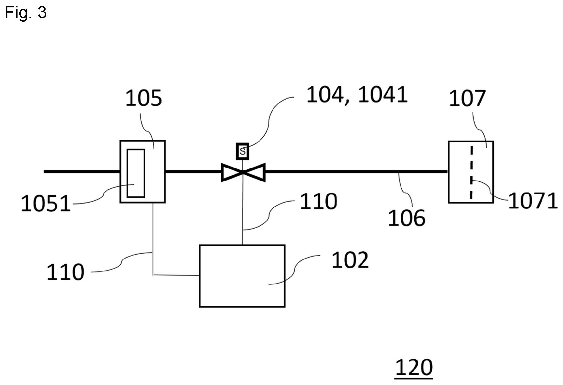

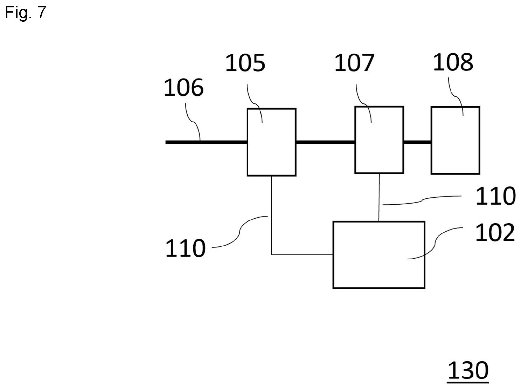

Fig. 3 zeigt schematisch einen beispielhaften, nicht beschränkenden Aufbau eines erfindungsgemäßen Systems (120) mit Absperrorgan (104), hier als Magnetventil (1041) ausgebildet. Zu sehen ist eine Vorbehandlungseinheit (105), welche mindestens einen Enthärter (1051) und einen Solebehälter mit daran angeordnetem Sensor (nicht dargestellt) umfasst. Das Magnetventil (1041) ist hinter der Vorbehandlungseinheit (105) so an einer Wasserleitung (106) angeordnet, dass es bei einer unzureichenden Regeneration des lonenaustausch-Materials im Enthärter (1051) (insbesondere wegen zu geringer Salzmenge im Solebehälter) den Wasserzulauf von der Vorbehandlungseinheit (105) zu dahinterliegenden Geräten, wie der hier gezeigten Umkehrosmoseeinheit (107), abschneiden kann. Die Umkehrosmoseeinheit (107) umfasst eine Membran (1071) für die Umkehrosmose. Das Abschneiden des Wasserzulaufs bei unzureichender Regeneration kann die Umkehrosmoseeinheit (107), insbesondere deren Membran (1071), vor Beschädigung schützen. In dem hier dargestellten Beispiel erfolgt hierzu zunächst eine Kommunikation (110) zwischen der Vorbehandlungseinheit (105) und der Auswerteeinheit (102). Diese kann dann bei Bedarf das Magnetventil (1041) ansteuern und schließen. Es ist so auch möglich, die Weichwasserzufuhr von der Vorbehandlungseinheit (105) an die Umkehrosmoseeinheit (107) während eines Regenerationsprozesses zu unterbrechen, sollte dies aus beispielsweise regulatorischer Sicht von Vorteil sein. Das in der Fig. 7 dargestellte System (120) kann optional auch die Umkehrosmoseeinheit (107) umfassen. Zusätzlich oder alternativ kann das Absperrorgan (104) auch direkt von der Vorbehandlungseinheit (105) angesteuert werden (hier nicht gezeigt).

### B.1.6. Kommunikationsmittel und Kommunikationsanbindung

Elemente des erfindungsgemäßen Systems (beispielsweise der Sensor und die Auswerteeinheit) können mit einem Kommunikationsmittel verbindbar oder verbunden sein und so kommunizieren, wie auch oben für das Verfahren und der vorstehenden Diskussion des Systems schon beschrieben. Entsprechende Kommunikationsmittel können Teil des Systems sein. Geeignete Kommunikationsmittel sind dem Fachmann bekannt und können von diesem bedarfsgerecht ausgewählt werden. Das Kommunikationsmittel kann eine drahtlose oder drahtgebundene Verbindung sein. Das Kommunikationsmittel kann bidirektional ausgestaltet sein. Es kann als Mittel zur digitalen Kommunikation, beispielsweise als Datenübertragungssystem oder Buskommunikation, ausgebildet sein.

Gemäß einer weiteren Ausführungsform umfasst das erfindungsgemäße System eine durch ein Kommunikationsmittel vermittelte Kommunikationsanbindung mit mindestens einem weiteren, insbesondere ein dem System nachgelagerten, Gerät.

Gemäß einer Ausführungsform kann die Auswerteinheit (102) mit mindestens einem nachgelagerten Gerät, insbesondere einer Umkehrosmoseeinheit (107), über ein Kommunikationsmittel (110) verbindbar oder verbunden sein, wobei das Kommunikationsmittel (110) bevorzugt als Mittel zur digitalen Kommunikation (110), beispielsweise als Datenübertragungssystem (1101) oder Buskommunikation (1102), ausgebildet ist. Die Umkehrosmoseeinheit (107) ist dabei bevorzugt ebenfalls Teil des erfindungsgemäßen Systems.

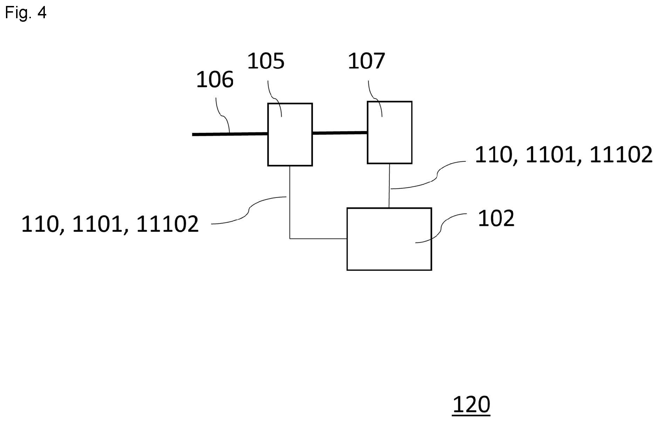

Fig. 4 zeigt schematisch einen beispielhaften, nicht beschränkenden Aufbau eines erfindungsgemäßen Systems (120). Zu sehen ist eine Vorbehandlungseinheit (105) und eine in Flussrichtung des durch eine Wasserleitung (106) fließenden Wassers nachgelagerte Umkehrosmoseeinheit (107) (Membran nicht dargestellt), sowie eine Auswerteeinheit (102). Die Auswerteeinheit (102) steht mit der Vorbehandlungseinheit (105) und der nachgelagerten Umkehrosmoseeinheit (107) über ein Kommunikationsmittel (110) in Kommunikationsverbindung. Die Auswerteeinheit (102) kann beispielsweise über ein Datenübertragungssystem (1101) oder Buskommunikation (1102) mit mindestens der Umkehrosmoseeinheit (107), kommunizieren. Die Kommunikation kann je nach Ausgestaltung eine Menge an Informationen übermitteln (wie Regenerationsprozess aktiv, Vorhandenes Salzgewicht zu Beginn der Regeneration, Regenerationsprozess nicht erfolgreich, unzureichend Verbleibende Regenerationszyklen bis Salz aufgebraucht). Aus den Informationen kann die Umkehrosmoseeinheit (107) Aktionen starten (wie Hartwassernotbetrieb, Anlagenstart bei aktiver Regeneration verschieben, Anzeigen von Daten etc.).

### B.1.7. Temperatursensor

Das System kann zusätzlich zu dem mindestens einen Sensor, insbesondere Drucksensor, einen Temperatursensor, beispielsweise ein Thermostat, zur Temperaturmessung im, am, oder in der Nähe des Solebehälters umfassen. Erfolgt eine Messung in der Nähe des Solebehälters, so wird vorzugsweise im selben Raum und/oder in einem Abstand von höchstens 5 m, 3 m oder 1 m zum Solebehälter gemessen. Der Temperatursensor kann mit der Auswerteinheit über ein Kommunikationsmittel verbindbar oder verbunden sein, und so die gemessene Temperatur an die Auswerteeinheit übermitteln. Das Kommunikationsmittel ist dabei bevorzugt als Mittel zur digitalen Kommunikation, beispielsweise als Datenübertragungssystem oder Buskommunikation, ausgebildet. Vorteilhaft kann in der Ausführungsform mit Temperatursensor die gemessene Temperatur zur Korrektur und/oder Auswahl der Dichte ρ_{Wasser} einer wässrigen Flüssigkeit im Solebehälter verwendet werden. Es wird hier insbesondere auch auf die obige Diskussion der Temperatur im Abschnitt zum theoretischen Wasserdruck (A.1.3.1. a)) verwiesen.

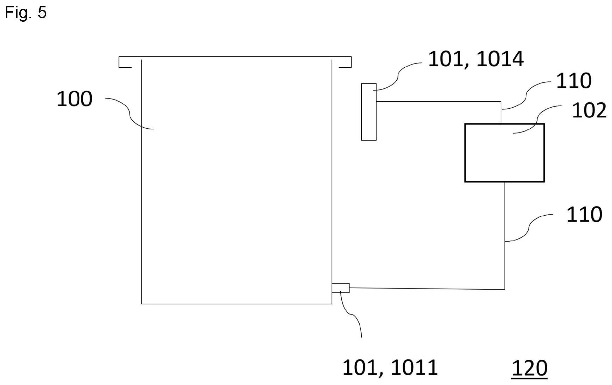

Fig. 5 zeigt schematisch einen beispielhaften, nicht beschränkenden Aufbau eines erfindungsgemäßen Systems (120) mit am Solebehälter (100) angeordnetem Drucksensor (101, 1011). Das System umfasst weiter einen in der Nähe des Solebehälters (100) angeordneten Temperatursensor (101, 1014), der ebenso wie der Drucksensor (101, 1011), mit der Auswerteeinheit (102) über ein Kommunikationsmittel (110) verbunden ist.

### B.2. System mit Füllstandsensor

Das System kann zusätzlich oder alternativ zu den vorbeschriebenen druckmessungsbasierten Ausführungsformen auch mindestens einen Füllstandssensor umfassen. Die Ausführungsform mit Füllstandsensor ist frei mit einer oder mehreren der vorbeschriebenen Ausführungsformen des Systems kombinierbar.

Die obige Beschreibung des Systems ist auch für die Ausführungsform mit Füllstandssensor anwendbar, sofern sich nicht aus dem Kontext etwas anderes ergibt. Die Ausführungen zum Füllstandsensor bei der Beschreibung des erfindungsgemäßen Verfahrens (vgl. A.2.) gelten entsprechend.

Gemäß einer Ausführungsform umfasst das System einen Füllstandssensor, der zum Messen eines Wasserfüllstandes am oder im, bevorzugt im, Solebehälter angeordnet ist.

Der Füllstandssensor kann zur Überwachung eines Auslösefüllstandes im Solebehälter angeordnet sein. Dabei kann der Auslösefüllstand ein Mindestfüllstand sein. Die Positionierung des Auslösefüllstands im Solebehälter kann dem Wasserfüllstand im Solebehälter entsprechen, den ein Einfüllvolumen in Gegenwart einer Salzmasse im Solebehälter aufweist, die für eine vorbestimmte Anzahl an Regenerationen ausreicht, bevorzugt für eine, zwei oder drei Regenerationen, am meisten bevorzugt für eine Regeneration.

Der Füllstandsensor kann mit weiteren Elementen des Systems über ein Kommunikationsmittel verbindbar oder verbunden sein, insbesondere mit einer Auswerteinheit und/oder einem Signalgeber. Das Kommunikationsmittel ist bevorzugt als Mittel zur digitalen Kommunikation, beispielsweise als Datenübertragungssystem oder Buskommunikation, ausgebildet.

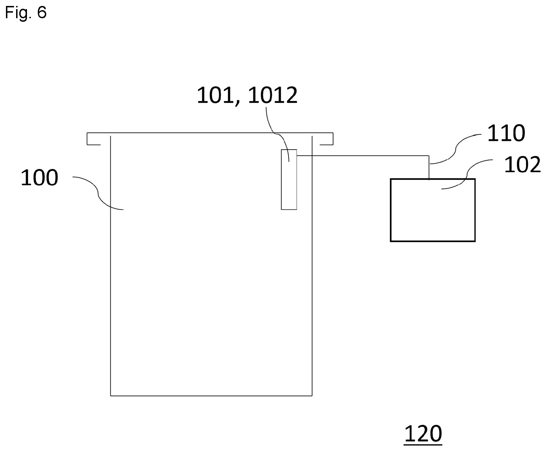

Fig. 6 zeigt schematisch einen beispielhaften, nicht beschränkenden Aufbau eines erfindungsgemäßen Systems (120) mit im Solebehälter (100) angeordnetem Füllstandssensor (101, 1012), der mit der Auswerteeinheit (102) über ein Kommunikationsmittel (110) verbunden ist.

### C. UMKEHROSMOSEANLAGE

Die Erfindung stellt in einem dritten Aspekt auch eine Umkehrosmoseanlage bereit. Die erfindungsgemäße Umkehrosmoseanlage ist dadurch gekennzeichnet, dass sie ein System gemäß des zweiten Aspekts der Erfindung umfasst. Auf die in der obigen Beschreibung und/oder in den Ansprüchen offenbarten Merkmale des erfindungsgemäßen Systems des zweiten Aspekts wird hierbei Bezug genommen. Die Offenbarung gilt entsprechend auch für die Umkehrosmoseanlage des dritten Aspekts und ist auf diese anwendbar.

Die Umkehrosmoseanlage kann optional weiter einen Permeatspeicher zur Speicherung von durch die Umkehrosmoseeinheit bereitgestelltem Permeat umfassen.

In einer bevorzugten Ausführungsform ist das von der Umkehrosmoseanlage umfasste System so ausgestaltet, dass es den Solebehälter (100) umfasst, und weiter
- eine Vorbehandlungseinheit (105) für eine Umkehrosmoseeinheit (107), die Vorbehandlungseinheit (105) umfassend mindestens einen ein Ionenaustauscher-Material umfassenden Enthärter (1051), und
- optional eine eine Membran (1071) umfassende Umkehrosmoseeinheit (107), die mit der Vorbehandlungseinheit (105) zur Zufuhr von vorbehandeltem Wasser an die Umkehrosmoseeinheit (107) verbindbar oder verbunden ist,
wobei optional der Solebehälter (100) Teil der Vorbehandlungseinheit (105) ist und/oder mit dem Enthärter (1051) der Vorbehandlungseinheit (105) zur Regeneration des Ionenaustauscher-Materials verbindbar oder verbunden ist.

Es ist bevorzugt, dass das in der Umkehrosmoseanlage umfasste System ein druckmessungsbasiertes System ist, welches einen Drucksensor und bevorzugt auch eine Auswerteeinheit umfasst.

Bei der Umkehrosmoseanlage kann es sich insbesondere um eine Umkehrosmoseanlage zur Aufbereitung von Wasser für die Dialyse, insbesondere die Hämodialyse, handeln. Die Umkehrosmoseanlage ist entsprechend in der Lage, hinreichend reines Wasser für die Dialyse zu liefern. Da das Wasser direkt mit dem Kreislauf des Patienten in Kontakt gelangt, wird eine hohe Anforderung an die Reinheit gestellt.

Fig. 7 zeigt schematisch einen beispielhaften, nicht beschränkenden Aufbau einer erfindungsgemäßen Umkehrosmoseanlage (130). Diese umfasst eine Vorbehandlungseinheit (105), eine damit über eine Wasserleitung (106) verbundene Umkehrosmoseeinheit (107) mit Membran (Membran nicht gezeigt), eine Auswerteeinheit (102) sowie in der hier gezeigten Ausführungsform einen Permeatspeicher (108). Dieser ist optional. Die Vorbehandlungseinheit (105) umfasst einen Solebehälter, bevorzugt mit Drucksensor (Solebehälter und Drucksensor sind aus Gründen der Übersichtlichkeit in Fig. 7 nicht gesondert dargestellt, vgl. hierzu beispielsweise Fig. 1B). Die Auswerteeinheit (102) ist mit der Vorbehandlungseinheit (105), insbesondere dem darin umfassten Sensor beziehungsweise Drucksensor, und mit der Umkehrosmoseeinheit (107) über ein Kommunikationsmittel verbunden (110).

### D. VERWENDUNGEN

In einem vierten Aspekt werden die folgenden erfindungsgemäßen Verwendungen bereitgestellt. Auf die in der obigen Beschreibung und/oder in den Ansprüchen offenbarten Merkmale des Verfahrens des ersten Aspekts, des Systems des zweiten Aspekts und der Umkehrosmoseanlage des dritten Aspekts wird hierbei Bezug genommen. Die Offenbarung ist entsprechend auch für die Verwendungen anwendbar.

Bereitgestellt wird die Verwendung eines Drucksensors zur Überwachung einer Salzfüllung eines Solebehälters einer Umkehrosmoseanlage. Der Drucksensor kann insbesondere eine oder mehrere der hierin, insbesondere bei der Diskussion des Systems gemäß des zweiten Aspekts, für den Drucksensor beschriebenen Eigenschaften aufweisen. Die vorstehenden Erklärungen zum Drucksensor sind anwendbar. Dementsprechend kann der Drucksensor insbesondere eine der vorbeschriebenen Eigenschaften des Drucksensors eines Systems des zweiten Aspekts der Erfindung aufweisen. Die Verwendung kann insbesondere im Rahmen der Aufreinigung von Wasser für ein Nierenersatzverfahren, bevorzugt für die Dialyse, am meisten bevorzugt die Hämodialyse, erfolgen.

Weiter bereitgestellt wird die Verwendung eines vorstehend und/oder in den Ansprüchen beschriebenen Systems gemäß des zweiten Aspekts zur Überwachung einer Salzfüllung eines Solebehälters einer Umkehrosmoseanlage. Die Verwendung kann insbesondere im Rahmen der Aufreinigung von Wasser für ein Nierenersatzverfahren, bevorzugt für die Dialyse, am meisten bevorzugt die Hämodialyse, erfolgen.

Auch bereitgestellt wird die Verwendung eines vorstehend und/oder in den Ansprüchen beschriebenen Systems gemäß des zweiten Aspekts der Erfindung oder der vorstehend und/oder in den Ansprüchen beschriebenen Umkehrosmoseanlage gemäß des dritten Aspekts der Erfindung im erfindungsgemäßen Verfahren des ersten Aspekts der Erfindung.

Zudem bereitgestellt wird Verwendung der Umkehrosmoseanlage des dritten Aspekts der Erfindung für die Wasseraufreinigung für ein Nierenersatzverfahren, bevorzugt für die Dialyse, am meisten bevorzugt die Hämodialyse.

### Beispiele

### Beispiel 1

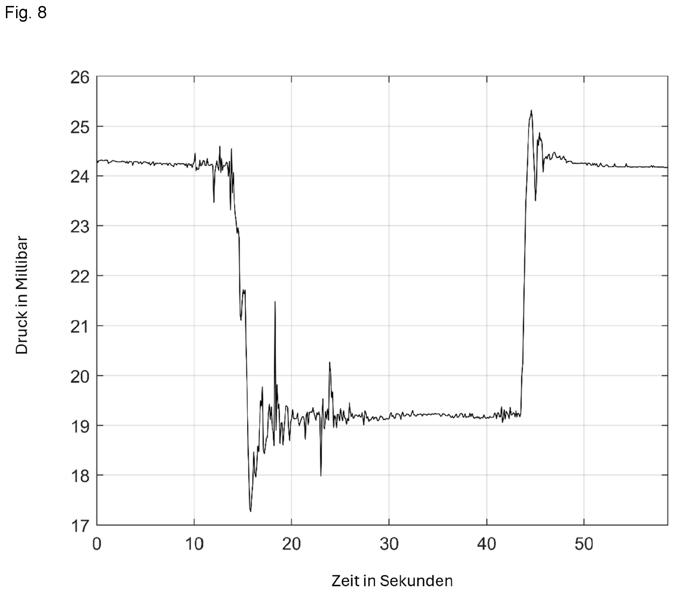

Ein in einem Solebehälter (Behälterradius= 0,265 m, Behältergrundfläche=0,2206 m²) befindlicher Salzsack mit Salztabletten wurde aus der im Solebehälter befindlichen gesättigten Salzlösung herausgenommen und dann wieder hineingetaucht. Dabei wurde der Druck der Salzlösung im Solebehälter mittels eines Drucksensors gemessen. Der Drucksensor war hierbei etwa 4 cm vom Unterboden des Solebehälters entfernt seitlich am Solebehälter derart angebracht, dass der Druck der im Solebehälter befindlichen Salzlösung gemessen werden konnte. Das Ergebnis der Druckmessung ist in Fig. 8 gezeigt. Der Druck der Salzlösung betrug:
- Druck mit Salzsack = 24,2 mBar = 2420 Pa = P_{Drucksensor}
- Druck ohne Salzsack = 19,3 mBar = 1930 Pa = P_{Wasserdruck(theoretisch)}

Hieraus wurde die Masse des Salzes m_{Salz} im Salzsack berechnet, unter Berücksichtigung folgender Werte:
- Dichte Salzwasser = 1202 kg/m³
- Dichte Salz = 2161,5 kg/m³
- Behälterradius= 0,265 m
- Behälterfläche=0,2206 m²
${P}_{Wasserverdr ä ngung} = {P}_{Drucksensor} - {P}_{Wasserdruck \left(Theoretisch\right)} = 24.2 E 2 Pa - 19.3 E 2 Pa = 480 Pa$ ${P}_{Wasserverdr ä ngung} = \frac{{P}_{Wasserverdr ä ngung} ∗ {A}_{Solebeh ä lter}}{{ρ}_{Salzwasser} ∗ g} = \frac{480 Pa ∗ 0.2206 {m}^{2}}{1202 \frac{kg}{{m}^{3}} ∗ 9.81 \frac{m}{{s}^{2}}} = 0.009 {m}^{3}$ ${V}_{Salz} = {V}_{Wasserverdr ä gung}$ ${m}_{Salz} = {ρ}_{Salz} ∗ {V}_{Salz} = 2161.5 \frac{kg}{{m}^{3}} ∗ 0.009 {m}^{3} = 19.4 kg$

Die berechnete Salzmasse wurde mit der durch Wiegen des Salzsacks ermittelten Masse (18,6 kg) verglichen. Die mittels Druckmessung bestimmte Masse entsprach mit einer Abweichung von < 5% mit guter Genauigkeit der Realität.

### Beispiel 2

Der Druck in einem Solebehälter (Behälterradius= 0,265 m, Behältergrundfläche=0,2206 m²) während einer Regeneration wurde mittels eines Drucksensors gemessen.

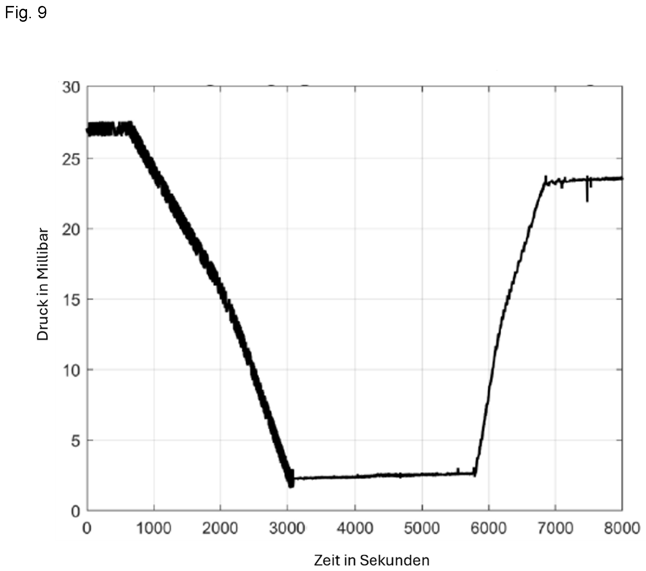

Das Ergebnis der Druckmessung ist in Fig. 9 gezeigt. In Fig. 9 links ist der Druck vor Beginn der Regeneration zu sehen. Während der Flüssigkeitsentnahme (gesättigte Salzlösung) zur Regeneration eines Ionentauschers kam es wie erwartet zur Druckabnahme. Über einen Zeitraum (3000s - 5700s) hatte der mit Abstand über dem Boden des Solebehälters eingebaute Sensor kein Signal, da der Flüssigkeitsspiegel unterhalb des Sensors lag (dies stellte in der Praxis kein Problem dar). Der Druck stieg dann mit Neubefüllung des Solebehälters mit Wasser für nächste Regeneration wieder an. Der Messwert schwankte vor der Regeneration etwas, was die Messung aber nicht beeinträchtigte. Die Schwankung wurde möglicherweise durch Vibrationen aufgrund der Spülung der Enthärter zu Beginn des Regenerationsprozesses verursacht.

Der Druck vor Beginn der Regeneration wurde genutzt, um die Salzmasse im Solebehälter vor der Regeneration zu ermitteln. Der Druck nach Neubefüllung wurde genutzt, um die Salzmasse im Solebehälter nach der Regeneration zu ermitteln.

Bei der Messung war ein Wasserdruck von 19.3698 mBar vorhanden. Im Ergebnis befanden sich vor der Regeneration (angesetzte Dichte = 1202 kg/m³) -31.4 kg Salz im Solebehälter. Nach der Regeneration (angesetzte Dichte = 998,2 kg/m³) befanden sich noch 20,8 kg Salz im Solebehälter. Diese Werte stimmen mit den erwarteten ~8kg Salzbedarf pro Regeneration überein.

### Beispiel 3

Wie oben dargelegt kann es zweckmäßig sein, ein von der Druckmessung nicht erfasstes Volumen zu berücksichtigen, wenn der Drucksensor oberhalb des Unterbodens des Solebehälters angeordnet ist. Dies wird an folgendem Beispiel weiter erläutert:
- Es wird davon ausgegangen, dass der Drucksensor den Druck 3 cm über dem Tankboden und somit dem Unterboden des Solebehälters misst.
- Der Tank hat eine Grundfläche von 0,2206 m² und wird mit einem Wasservolumen von 45 Liter befüllt. Die ersten 6,6 Liter führen in einem Tank ohne Salz bzw. bei dem das Salz bspw. wie in Beispiel 1 in einem Salzsack enthalten ist, der im Tank hängt, zu keiner Druckänderung.
- Entsprechend werden beispielsweise in der Gleichung für P_{Wasserdruck(theoretisch)} die 6,6 Liter von V_{Wasser} abgezogen.

### Bezugszeichenliste

100 - Solebehälter
   1001 - Unterboden
   1002 - Seitenwand
   1003 - Innenraum
   1004 - Öffnung
   1005 - Deckel
   1006 - Salzträger
101 - Sensor
   1011 - Drucksensor
   1012 - Füllstandssensor
   1013 - Volumenstromsensor
   1014 - Temperatursensor
102 - Auswerteeinheit
103 - Signalgeber
104 - Absperrorgan
   1041 - Magnetventil
105 - Vorbehandlungseinheit
   1051 - Enthärter
106 - Wasserleitung
   1061 - Wasserzulauf
107 - Umkehrosmoseeinheit
   1071 - Membran
108 - Permeatspeicher
109 - Soleleitung
110 - Kommunikationsmittel
   1101 - Datenübertragungssystem
   1102 - Buskommunikation
120 - System
130 - Umkehrosmoseanlage

## Patentansprüche

1. Verfahren zur Überwachung einer Salzfüllung eines Solebehälters (100) einer Umkehrosmoseanlage (130), wobei das Verfahren umfasst:
- Messen mindestens eines Messwertes mindestens einer Messgröße einer wässrigen Flüssigkeit im Solebehälter (100) mittels mindestens eines Sensors (101),
- Übermitteln des mindestens einen Messwerts an mindestens eine Auswerteeinheit (102), und
- Verarbeiten des mindestens einen Messwertes in der mindestens einen Auswerteeinheit (102), wobei der Sensor (101) ein Drucksensor (1011) ist, und das Verfahren umfasst:
- Messen eines Wasserdrucks P_{Drucksensor} der wässrigen Flüssigkeit im Solebehälter (100) mittels des Drucksensors (1011),
- Übermitteln des Wasserdrucks P_{Drucksensor} an die Auswerteeinheit (102), und
- Verarbeiten des Wasserdrucks P_{Drucksensor} in der Auswerteeinheit (102),
wobei das Verarbeiten des Wasserdrucks P_{Drucksensor} die Berechnung eines Wasserverdrängungsdrucks P_{Wasserverdrängung} umfasst, zur Bestimmung einer Salzfüllung des Solebehälters (100).

2. Verfahren nach Anspruch 1, wobei der Wasserverdrängungsdruck P_{Wasserverdrängung} wie folgt berechnet wird:
(A) mittels der Gleichung ${p}_{Wasserverdrägung} \left[\frac{kg}{{s}^{2} ∗ m}\right] = {p}_{Drucksensor} \left[\frac{kg}{{s}^{2} ∗ m}\right] - {p}_{wasserdruck \left(theoretisch\right)} \left[\frac{kg}{{s}^{2} ∗ m}\right] ,$ mit ${p}_{wasserdruck \left(theoretisch\right)} \left[\frac{kg}{{s}^{2} ∗ m}\right] = \frac{{ρ}_{wasser} \left[\frac{kg}{{m}^{3}}\right] ∗ {V}_{wasser} \left[{m}^{3}\right] ∗ g \left[\frac{m}{{s}^{2}}\right]}{{A}_{Solebeh ä lter \left[{m}^{2}\right]}} ,$ wobei ρWasser eine Dichte der wässrigen Flüssigkeit, A_{Solebehälter} die Grundfläche des Solebehälters (100), g die Erdbeschleunigung und V_{Wasser} ein festgelegter Wasservolumenwert ist, wobei der Wasservolumenwert zumindest im Wesentlichen dem Wasservolumen entspricht, mit dem der Solebehälter (100) bei der Messung des Wasserdrucks P_{Drucksensor} befüllt ist, oder
(B) an einem Wasserzulauf (1061) des Solebehälters (100) befindet sich ein Volumenstromsensor (1013), der bei der Befüllung des Solebehälters (100) mit Wasser das zugeführte Wasservolumen misst und zur Ermittlung des Wasserdrucks P_{Wasserdruck} an die Auswerteeinheit (102) übermittelt, und der Wasserverdrängungsdruck P_{Wasserverdrängung} wird berechnet mittels der Gleichung ${p}_{Wasserverdr ä ngung} \left[\frac{kg}{{s}^{2} ∗ m}\right] = {p}_{Drucksensor} \left[\frac{kg}{{s}^{2} ∗ m}\right] - {p}_{wasserdruck} \left[\frac{kg}{{s}^{2} ∗ m}\right] ,$ mit ${p}_{wasserdruck} \left[\frac{kg}{{s}^{2} ∗ m}\right] = \frac{{p}_{Wasser} \left[\frac{kg}{{m}^{3}}\right] ∗ {V}_{wasser} \left(gemessen\right) \left[{m}^{2}\right] ∗ g \left[\frac{m}{{s}^{2}}\right]}{{A}_{Solebeh ä lter \left[{m}^{2}\right]}} ,$ wobei ρWasser eine Dichte der wässrigen Flüssigkeit, A_{Solebehälter} die Grundfläche des Solebehälters (100), g die Erdbeschleunigung und V_{Wasser (gemessen)} der vom Volumenstromsensor (1013) bei der Befüllung des Solebehälters (100) mit Wasser gemessene Wasservolumenwert ist, wobei das Wasservolumen, mit dem der Solebehälter (100) bei der Messung des Wasserdrucks P_{Drucksensor} befüllt ist, zumindest im Wesentlichen dem gemessenen Wasservolumenwert entspricht.

3. Verfahren nach Anspruch 2, **dadurch gekennzeichnet, dass** das Verfahren weiter umfasst:
- Vergleichen des berechneten Wasserverdrängungsdrucks P_{Wasserverdrängung} mit einem Druckschwellenwert für den Wasserverdrängungsdruck P_{Wasserverdrängung}, und
- Auslösen eines Signals, wenn der berechnete Wasserverdrängungsdruck gleich dem oder kleiner als der Druckschwellenwert ist,
wobei optional
(i) der Druckschwellenwert für eine Mindestsalzfüllung des Solebehälter (100) repräsentativ ist,
(ii) der Druckschwellenwert dem Druck entspricht, den ein Einfüllwasservolumen in Gegenwart einer Salzmasse im Solebehälter (100) aufweist, die für eine vorbestimmte Anzahl an Regenerationen, bevorzugt eine Regeneration, eines Ionenaustauscher-Materials eines Enthärters (1051) einer Vorbehandlungseinheit (105) ausreicht, wobei das Einfüllwasservolumen bevorzugt dem Wasservolumen entspricht, mit dem der Solebehälter (100) zur Herstellung einer Salzlösung für die Regeneration befüllt wird, wenn er noch keine Flüssigkeit enthält,
(iii) der Vergleich des berechneten Wasserverdrängungsdrucks mit dem Druckschwellenwert in der Auswerteinheit (102) erfolgt, bevorzugt wobei der Druckschwellenwert in der Auswerteeinheit (102) hinterlegt ist,
(iv) das Auslösen des Signals selbsttätig erfolgt, und/oder das Signal ausgewählt ist aus einem akustischen Signal, einem optischen Signal, einem als Alarm ausgestalten Signal, einer Salznachfüllaufforderung, oder Kombinationen davon, und/oder
(v) das Signal ein Absperrorgan (104), bevorzugt ein Magnetventil (1041), ansteuert, um den Wasserzulauf von einer Vorbehandlungseinheit (105) in Richtung dahinterliegender Geräte, wie einer Umkehrosmoseeinheit (107), abzuschneiden.

4. Verfahren nach Anspruch 2 oder 3, **dadurch gekennzeichnet, dass** das Verarbeiten des Wasserdrucks P_{Drucksensor} umfasst:
(A') Berechnen der im Solebehälter (100) befindlichen Salzmasse m_{Salz} mittels der Gleichung ${m}_{Salz} = {ρ}_{Salz} ∗ \frac{\left({p}_{Drucksensor}-{p}_{wasserdruck \left(theoretisch\right)}\right) ∗ {A}_{Solebeh ä lter}}{{ρ}_{Wasser} ∗ g} ,$ wobei P_{Wasserdruck(theoretisch)} wie in der optionalen Ausführungsform (A) von Anspruch 3 beschrieben definiert ist,
oder
(B') an einem Wasserzulauf (1061) des Solebehälters (100) befindet sich ein Volumenstromsensor (1013), der bei der Befüllung des Solebehälters (100) mit Wasser das zugeführte Wasservolumen misst und zur Ermittlung des Wasserdrucks P_{Wasserdruck} an die Auswerteeinheit (102) übermittelt, und die im Solebehälter (100) befindlichen Salzmasse m_{Salz} wird berechnet mittels der Gleichung ${m}_{Salz} = {ρ}_{Salz} ∗ \frac{\left({p}_{Drucksensor}-{p}_{wasserdruck}\right) ∗ {A}_{Solebeh ä lter}}{{ρ}_{Wasser} ∗ g} ,$ wobei P_{Wasserdruck} wie in der optionalen Ausführungsform (B) von Anspruch 3 beschrieben definiert ist.

5. Verfahren nach Anspruch 4, **dadurch gekennzeichnet, dass** das Verfahren weiter umfasst:
- Messen der Anzahl der Regenerationen, die mit der im Solebehälter (100) befindlichen Salzmasse möglich sind,
- Messen einer nachzufüllenden Salzmasse, und/oder
- Vergleichen der berechneten Salzmasse mit einem Salzmassenschwellenwert, und Auslösen eines Signals, wenn die berechnete Salzmasse gleich dem oder kleiner als der Salzmassenschwellenwert ist, wobei optional
(i) der Salzmassenschwellenwert für eine Mindestsalzmasse in dem Solebehälter (100) repräsentativ ist,
(ii) der Salzmassenschwellenwert einer Salzmasse im Solebehälter (100) entspricht, die für eine vorbestimmte Anzahl an Regenerationen, bevorzugt eine Regeneration, eines Ionenaustauscher-Materials eines Enthärters (1051) einer Vorbehandlungseinheit (105) ausreicht,
(iii) der Vergleich der berechnete Salzmasse mit dem Salzmassenschwellenwert in der Auswerteinheit (102) erfolgt, bevorzugt wobei der Salzmassenschwellenwert in der Auswerteeinheit (102) hinterlegt ist,
(iv) das Auslösen des Signals selbsttätig erfolgt, und/oder das Signal ausgewählt ist aus einem akustischen Signal, einem optischen Signal, einem als Alarm ausgestalteten Signal, einer Salznachfüllaufforderung, oder Kombinationen davon, und/oder
(v) das Signal ein Absperrorgan (104), bevorzugt ein Magnetventil (1041), ansteuert, um den Wasserzulauf von einer Vorbehandlungseinheit (105) in Richtung dahinterliegender Geräte, wie einer Umkehrosmoseeinheit (107), abzuschneiden.

6. Verfahren nach einem der Ansprüche 2 bis 5, **dadurch gekennzeichnet, dass**
a) die Dichte der wässrigen Flüssigkeit ρ_{Wasser} in Abhängigkeit von der Verweildauer des Wassers im Solebehälter (100) vor der Druckmessung ausgewählt wird, optional wobei
(i) als ρ_{Wasser} die Dichte reinen Wassers, bevorzugt 998,2 kg/m³, ausgewählt wird, wenn die Druckmessung innerhalb von 0,5 h, bevorzugt innerhalb von 0,25 h, weiter bevorzugt innerhalb von 5 min oder unmittelbar nach dem Befüllen des Solebehälters (100) mit einem Wasservolumen erfolgt, wenn der Solebehälter (100) vor dem Befüllen mit dem Wasservolumen im Wesentlichen keine oder keine Flüssigkeit enthielt, und/oder
(ii) als ρ_{Wasser} die Dichte einer gesättigten Salzlösung, bevorzugt 1202 kg/m³, ausgewählt wird, wenn die Druckmessung mindestens 1 h, bevorzugt mindestens 1,5 h, weiter bevorzugt mindestens 2 h nach dem Befüllen des Solebehälters (100) mit einem Wasservolumen erfolgt, wenn der Solebehälter (100) vor dem Befüllen mit dem Wasservolumen im Wesentlichen keine oder keine Flüssigkeit enthielt,
und/oder
b) der Einfluss der Temperatur auf die Dichte der wässrigen Flüssigkeit ρ_{Wasser} wie folgt berücksichtigt wird:
(i) eine Standarddichte wird als ρ_{Wasser} angenommen und der Temperatureinfluss auf die Dichte vernachlässigt,
(ii) es erfolgt eine Temperaturmessung im Solebehälter (100) und ρ_{Wasser} wird gemäß der gemessenen Temperatur korrigiert und/oder ausgewählt,
(iii) es erfolgt eine Temperaturmessung, beispielsweise mittels eines Thermostats, in der Nähe des Solebehälters (100) und ρ_{Wasser} wird gemäß der gemessenen Temperatur korrigiert und/oder ausgewählt, und/oder
(iv) in der Auswerteeinheit (102) ist eine durchschnittliche Temperatur, bevorzugt eine durchschnittliche Raumtemperatur, hinterlegt, die zur Kompensation und/oder Auswahl von ρ_{Wasser} verwendet wird,
wobei (i) bevorzugt ist.

7. Verfahren nach einem der Ansprüche 1-6, **dadurch gekennzeichnet, dass**
(i) ein oder mehrere Werte zur Berechnung von P_{Wasserdruck (theoretisch)} und/oder P_{Wasserdruck} in der Auswerteinheit (102), bevorzugt einem persistenten Speicher der Auswerteeinheit (102), hinterlegt sind, und/oder
(ii) die Auswerteinheit (102) mit mindestens einem nachgelagerten Gerät, insbesondere einer Umkehrosmoseeinheit (107), über ein Kommunikationsmittel (110) kommunizieren kann, wobei das Kommunikationsmittel (110) bevorzugt als Mittel zur digitalen Kommunikation (110), beispielsweise als Datenübertragungssystem (1101) oder Buskommunikation (1102), ausgebildet ist.

8. System (120) zur Überwachung einer Salzfüllung eines Solebehälters (100) für eine Umkehrosmoseanlage (130), wobei das System (120) mindestens einen Sensor (101) zum Messen mindestens eines Messwertes mindestens einer Messgröße einer gegebenenfalls im Solebehälter (100) enthaltenen wässrigen Flüssigkeit umfasst, wobei der mindestens einen Sensor (101) zum Messen des mindestens eines Messwertes am oder im Solebehälter (100) angeordnet ist, und wobei
- das System (120) weiter eine Auswerteeinheit (102) umfasst,
- das System (120) einen Drucksensor (1011) umfasst, der zum Messen eines Wasserdrucks P_{Drucksensor} der wässrigen Flüssigkeit am oder im Solebehälter (100) angeordnet ist,
- die Auswerteeinheit (102) dazu ausgebildet ist, den Wasserdruck P_{Drucksensor} zu verarbeiten und daraus einen Wasserverdrängungsdruck *P*_{Wasserverdrängun}g zu bestimmen und daraus eine Salzfüllung des Solebehälters (100) zu bestimmen.

9. System (120) nach Anspruch 8, **dadurch gekennzeichnet, dass** das System (120) mindestens eine der folgenden Eigenschaften hat:
(i) das System (120) umfasst einen Drucksensor (1011) und/oder oder ein Füllstandsensor (1012),
(ii) das System (120) umfasst einen Drucksensor (1011), der wenigstens in einem Teil, der mit der wässrigen Flüssigkeit in Kontakt gelangt, korrosionsresistent ist und/oder der eine bündig mit dem Drucksensorgehäuse abschließende Messmembran hat,
(iii) der mindestens eine Sensor (101), insbesondere Drucksensor (1011), ist am oder im Solebehälter (100) angebracht,
(iv) der mindestens eine Sensor (101), insbesondere Drucksensor (1011), ist mit dem Solebehälter (100) reversibel oder dauerhaft verbunden,
(v) der mindestens eine Sensor (101), insbesondere Drucksensor (1011), ist als integraler Bestandteil des Solebehälters (100) ausgebildet;
(vi) der mindestens eine Sensor (101), insbesondere Drucksensor (1011), ist mit der Auswerteeinheit (102) zur Übermittlung des Messwertes über ein Kommunikationsmittel (110) verbindbar oder verbunden, wobei das Kommunikationsmittel (110) bevorzugt als Mittel zur digitalen Kommunikation (110), beispielsweise als Datenübertragungssystem (1101) oder Buskommunikation (1102), ausgebildet ist.

10. System (120) nach Anspruch 8 oder 9, wobei
- der Drucksensor in der Nähe des Unterbodens (1001) des Solebehälters an einer Seitenwand (1002) des Solebehälters (100) angeordnet ist, insbesondere bis zu 10 cm, bis zu 8 cm, bis zu 5 cm, bis zu 4 cm oder etwa 3 cm vom Unterboden (1001) des Solebehälters (100) entfernt,
- der Drucksensor (1011) so angeordnet ist, dass er bei relativer Druckmessung einen Druckmesswert > 0 Pa liefert, wenn der Solebehälter mit einem Einfüllwasservolumen gefüllt ist und eine für eine Regeneration benötigte Mindestmasse an Salz enthält, wobei der Druckmesswert 0 Pa ist, wenn die Mindestmasse an Salz unterschritten ist,
- der Drucksensor (1011) so angeordnet ist, dass er bei relativer Druckmessung einen Druckmesswert > 0 Pa liefert, wenn der Solebehälter mit einem Einfüllwasservolumen gefüllt ist, der Behälter aber kein Salz enthält, wobei der Druckmesswert 0 Pa ist, wenn das Einfüllwasservolumen unterschritten ist, und/oder
- der Drucksensor (1011) am Unterboden (1001) des Solebehälters (100) angeordnet ist und die Druckmessung von unten durch den Unterboden (1001) erfolgt,
wobei (i) bevorzugt ist, und wobei das Einfüllwasservolumen bevorzugt dem Wasservolumen entspricht, mit dem der Solebehälter (100) zur Herstellung einer Salzlösung für die Regeneration befüllt wird, wenn er noch keine Flüssigkeit enthält.

11. System (120) nach einem der Ansprüche 8-10, **dadurch gekennzeichnet, dass** das System (120) mindestens eine Auswerteeinheit (102) umfasst, wobei optional
(i) die Auswerteinheit (102) als Teil des Sensors (101, 1011, 1012) ausgebildet ist,
(ii) die Auswerteinheit (102) mit dem Sensor (101, 1011, 1012) über ein Kommunikationsmittel (110) verbindbar oder verbunden ist,
(iii) die Auswerteinheit (102) der Verarbeitung des mindestens einen Messwertes dient,
(iv) die Auswerteinheit (102) über einen Speicher, bevorzugt einen persistenten Speicher, verfügt, in dem ein oder mehrere Werte, bevorzugt umfassend ein oder mehrere Werte zur Berechnung von P_{Wasserdruck} (theoretisch) und/oder P_{Wasserdruck}, hinterlegt sind, und/oder
(v) die Auswerteinheit (102) mit mindestens einem nachgelagerten Gerät, insbesondere einer Umkehrosmoseeinheit (107), über ein Kommunikationsmittel (110) verbindbar oder verbunden ist, wobei das Kommunikationsmittel (110) bevorzugt als Mittel zur digitalen Kommunikation (110), beispielsweise als Datenübertragungssystem (1101) oder Buskommunikation (1102), ausgebildet ist.

12. System (120) nach einem der Ansprüche 8-11, **dadurch gekennzeichnet, dass** das System (120) umfasst:
a) einen Volumenstromsensor (1013) zur Messung des bei der Befüllung des Solebehälters (100) zugeführten Wasservolumens V_{Wasser(gemessen)}, wobei optional
(i) der Volumenstromsensor (1013) mit der Auswerteinheit (102) über ein Kommunikationsmittel (110) verbindbar oder verbunden ist, und/oder
(ii) der Volumenstromsensor (1013) sich an einem Wasserzulauf (1061) des Solebehälters (100) befindet,
b) einen Signalgeber (103), wobei optional
(i) der Signalgeber (103) als Teil der Auswerteinheit (102) ausgebildet ist, und/oder
(ii) die Auswerteinheit (102) mit dem Signalgeber (103) über ein Kommunikationsmittel (110) verbindbar oder verbunden ist,
c) ein Absperrorgan (104), bevorzugt ein Magnetventil (1041), das mit der Auswerteeinheit (102) und/oder einer Vorbehandlungseinheit (105) über ein Kommunikationsmittel (110) verbindbar oder verbunden ist, insbesondere wobei das Absperrorgan (104) von der Auswerteeinheit (102) und/oder der Vorbehandlungseinheit (105) zur Unterbrechung einer Wasserzufuhr von der Vorbehandlungseinheit (105) in Richtung dahinterliegender Geräte, wie einer Umkehrosmoseeinheit (107), ansteuerbar ist, und/oder
d) eine durch ein Kommunikationsmittel (110) vermittelte Kommunikationsanbindung mit mindestens einem weiteren Gerät,
wobei in a) bis d) das Kommunikationsmittel (110) unabhängig voneinander gewählt sein kann, wobei das Kommunikationsmittel (110) bevorzugt als Mittel zur digitalen Kommunikation (110), beispielsweise als Datenübertragungssystem (1101) oder Buskommunikation (1102), ausgebildet ist.

13. System (120) nach einem der Ansprüche 8-12, wobei dass das System (120) den Solebehälter (100) umfasst, **dadurch gekennzeichnet, dass** das System (120) weiter umfasst:
- eine Vorbehandlungseinheit (105) für eine Umkehrosmoseeinheit (107), die Vorbehandlungseinheit (105) umfassend mindestens einen ein Ionenaustauscher-Material umfassenden Enthärter (1051), und
- optional eine eine Membran (1071) umfassende Umkehrosmoseeinheit (107), die mit der Vorbehandlungseinheit (105) zur Zufuhr von vorbehandeltem Wasser an die Umkehrosmoseeinheit (107) verbindbar oder verbunden ist,
wobei optional der Solebehälter (100) Teil der Vorbehandlungseinheit (105) ist und/oder mit dem Enthärter (1051) der Vorbehandlungseinheit (105) zur Regeneration des Ionenaustauscher-Materials verbindbar oder verbunden ist.

14. Umkehrosmoseanlage (130) umfassend ein System (120) nach einem der Ansprüche 8-13.
